(19) Europäisches Patentamt
European Patent Office
Office européen des brevets

(11) Veröffentlichungsnummer: **0 284 935**
**A1**

(12) **EUROPÄISCHE PATENTANMELDUNG**

(21) Anmeldenummer: **88104452.3**

(22) Anmeldetag: **21.03.88**

(51) Int. Cl.⁴ **C07D 215/56 , C07D 471/04 , C07D 498/06 , A61K 31/47 , A61K 31/44 , A61K 31/535 , //(C07D471/04,221:00,221:00), (C07D498/06,265:00,221:00)**

(30) Priorität: **02.04.87 DE 3711193**

(43) Veröffentlichungstag der Anmeldung: **05.10.88 Patentblatt 88/40**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Petersen, Uwe, Dr.**
**Auf dem Forst 4**
**D-5090 Leverkusen 1(DE)**
Erfinder: **Grohe, Klaus, Dr.**
**Am Wasserturm 10**
**D-5068 Odenthal(DE)**
Erfinder: **Schriewer, Michael, Dr.**
**Am Thelen Siefen 1a**
**D-5068 Odenthal(DE)**
Erfinder: **Schenke, Thomas, Dr.**
**Morgengraben 3**
**D-5000 Köln 80(DE)**
Erfinder: **Haller, Ingo, Dr.**
**Dornröschenweg 4**
**D-5600 Wuppertal 1(DE)**
Erfinder: **Metzger, Karl Georg, Dr.**
**Pahlkestrasse 75**
**D-5600 Wuppertal 1(DE)**
Erfinder: **Endermann, Rainer, Dr.**
**In den Birken 152a**
**D-5600 Wuppertal 1(DE)**
Erfinder: **Zeiler, Hans-Joachim, Dr.**
**Elsbeeker Strasse 46**
**D-5620 Velbert 15(DE)**

(54) **5-Substituierte Chinolon- und Naphthyridoncarbonsäure-Derivate.**

(57) Die Erfindung betrifft 5-substituierte Chinolon-und Naphthyridoncarbonsäure-Derivate der Formel (I)

EP 0 284 935 A1

Xerox Copy Centre

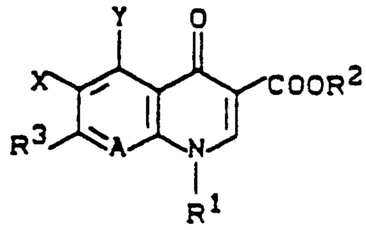

(I),

in welcher X, Y, R', $R^2$ und $R^3$ die in der Beschreibung angegebene Bedeutung haben, Verfahren zu ihrer Herstellung sowie diese enthaltende antibakterielle Mittel und Futterzusatzstoffe.

# 5-Substituierte Chinolon-und Naphthyridoncarbonsäure-Derivate

Die Erfindung betrifft 5-substituierte Chinolon-und Naphthyridoncarbonsäure-Derivate, Verfahren zu ihrer Herstellung sowie diese enthaltende antibakterielle Mittel und Futterzusatzstoffe.

Es ist bereits eine Reihe von Patentanmeldungen bekanntgeworden, in denen 5-Aminochinoloncarbonsäuren als antibakterielle Mittel beansprucht werden. Die japanische Patentanmeldung 57 149 286 beansprucht Pyridobenzoxazin-Derivate folgender Struktur (1),

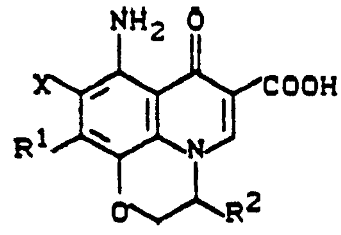

(1)

in welcher
R' für einen Aminorest und
$R^2$ für Wasserstoff oder Alkyl steht.

Die Einführung der Aminogruppe erfolgt dadurch, daß das Pyridobenzoxazin nitriert wird und die Nitrogruppe anschließend zur Aminogruppe reduziert wird.

Auch die in der europäischen Patentanmeldung 172 651, der südafrikanischen Patentanmeldung 8 502 369 und der japanischen Patentanmeldung 58 174 367 beanspruchten 5-Aminochinoloncarbonsäuren werden über 5-Nitrochinoloncarbonsäuren, die zunächst aus geeigneten Nitroaromaten synthetisiert werden müssen, hergestellt.

Es wurde jetzt gefunden, daß 5-substituierte Chinolon-und Naphthyridoncarbonsäure-Derivate der Formel (I)

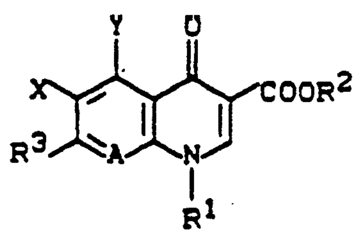

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatom je Alkylgruppe, Cycloalkylamino mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,
$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl und
$R^3$ für Methyl oder eine cyclische Aminogruppe wie

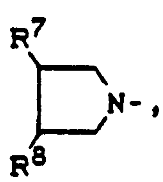 , 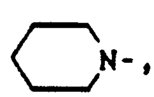 , 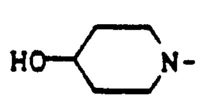 , 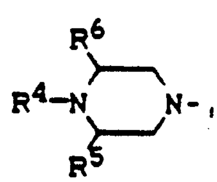 ,

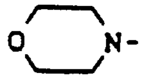, 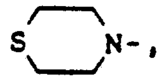, 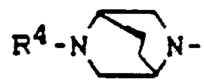, 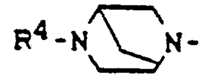,

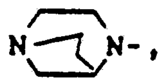, 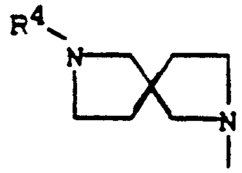, 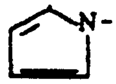, 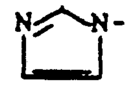,

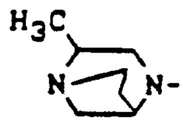

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze oder zugrundeliegenden Carbonsäuren eine hohe antibakterielle Wirkung insbesondere im grampositiven Bereich aufweisen, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydrochinolin-3-carbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspirol[4.4]non-2-yl und 7-Methyl (bzw. Ethyl)-2,7-diazaspiro[4.4]non-2-yl bedeutet, und 5-Amino-7-(3-amino-1-pyrrolidinyl)-1-ethyl-6-fluor-1,4-dihydro-4-oxo-1,8-naphthyridin-3-carbonsäure.

Sie eignen sich daher als Wirkstoffe für die Human-und Veterinärmedizin, wobei zur Veterinärmedizin auch die Behandlung von Fischen zur Therapie oder Vorbeugung bakterieller Infektionen zu zählen ist.

Bevorzugt sind 5-substituierte Chinolon-und Naphthyridoncarbonsäure-Derivate der Formel (I)

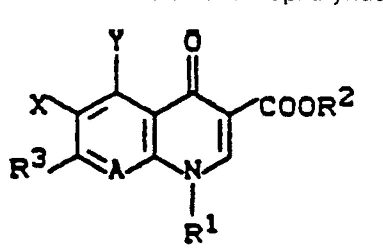

(I),

in welcher

X für Fluor oder Chlor,

Y für Amino, gegebenanfalls durch Amino, Hydroxy oder Methoxy substituiertes Alkylamino mit 1 bis 4 Kohlenstoffatomen, Dialkylamino mit 1 bis 4 Kohlenstoffatomen je Alkylgruppe, Cyclo alkylamino mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,

$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,

$R^2$ für Wasserstoff, Alkyl mit 1 bis 4 Kohlenstoffatomen oder (5-Methyl-2-oxo-1,3-dioxol-4-yl)-methyl und

$R^3$ für Methyl oder eine cyclische Aminogruppe wie

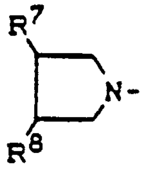,
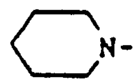,
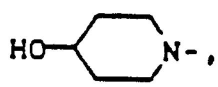,
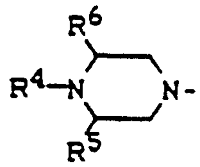,

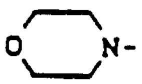,
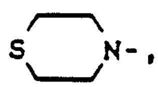,
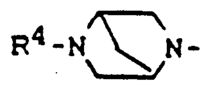,
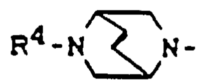,

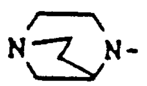,
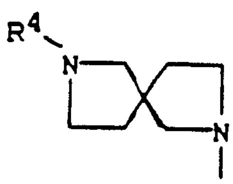,
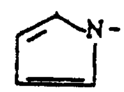,
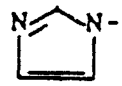,

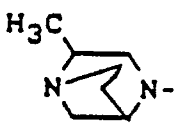

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze oder zugrundeliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspirol-

[4.4]non-2-yl und 7-Methyl (bzw. Ethyl)-2,7-diazaspiro[4.4]non-2-yl bedeutet, und 5-Amino-7-(3-amino-1-pyrrolidinyl)-1-ethyl-6-fluoro-1,4-dihydro-4-oxo-1,8-naphthyridincarbonsäure, sowie ausgenommen Verbindungen, in denen $R^3$ für den Rest

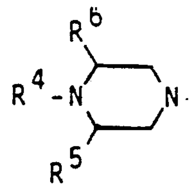

mit $R^4$, $R^5$ und $R^6$ = H oder $C_1$-$C_4$-Alkyl steht, wenn R' Cyclopropyl, X Fluor, Y Amino, Hydroxy oder $C_1$-$C_4$-Alkoxy und A N bedeutet, $R^9$ für Halogen oder H steht,
$R^2$ H bedeutet und deren Säureadditionssalze und Verbindungen, in denen Y für Amino oder Chlor steht, R' Cyclopropyl und X Fluor bedeutet, A für CF steht sowie $R^3$

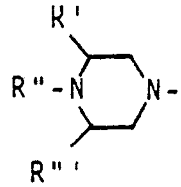

bedeutet,
worin R' H oder $CH_3$, R'' H, $CH_3$ oder $C_2H_5$, R''' H oder Methyl bedeuten, und $R^2$ für H, $CH_3$ oder $C_2H_5$ steht.

Bevorzugt sind auch diejenigen Verbindungen der Formel (I), in denen
X für Fluor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen je Alkylgruppe, Cyclopropylamino, Alkoxyamino mit 1 bis 3 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydroxyamino,
R' für Ethyl, Isopropyl, Cyclopropyl, Vinyl, 2-Hydroxyethyl, 2-Fluorethyl, Phenyl, 4-Fluorphenyl oder 2,4-Difluorphenyl,
$R^2$ für Wasserstoff, Alkyl mit 1 bis 3 Kohlenstoffatomen, (5-Methyl-2-oxo-1,3-dioxol-4-yl)-methyl,
$R^3$ für eine cyclische Aminogruppe wie

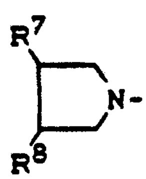, 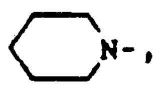, 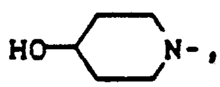, 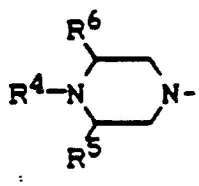,

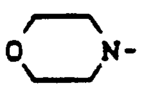, 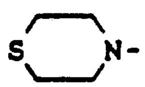, 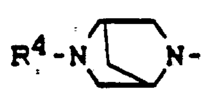, 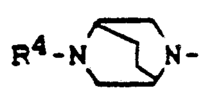,

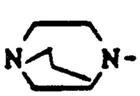, 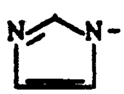, 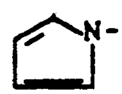, 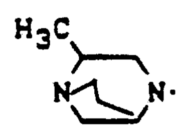

steht, worin
$R^4$ für Wasserstoff, Alkyl mit 1 bis 3 Kohlenstoffatomen, 2-Hydroxyethyl, Allyl, 2-Oxopropyl, 3-Oxobutyl, Phenacyl, Benzyl, 4-Aminobenzyl, (5-Methyl-2-oxo-1,3-dioxol-4-yl)-methyl,

$R^5$ für Wasserstoff oder Methyl,

$R^6$ für Wasserstoff, Alkyl mit 1 bis 2 Kohlenstoffatomen, Phenyl, 4-Fluorphenyl,

$R^7$ für Wasserstoff, Amino, Aminomethyl, Methylaminomethyl, Ethylaminomethyl, Dimethylaminomethyl, Hydroxy oder Hydroxymethyl,

$R^8$ für Wasserstoff oder Methyl steht,

A für N oder C-$R^9$ steht, worin

$R^9$ für Wasserstoff, Halogen wie Fluor oder Chlor steht oder auch gemeinsam mit R' eine Brücke der Struktur

$-O-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze oder zugrundeliegenden Carbonsäuren, ausgenommen 8-Amino-7-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl bedeutet und 5-Amino-7-(3-amino-1-pyrrolidinyl)-1-ethyl-6-fluor-1,4-dihydro-4-oxo-1,8-naphthyridin-3-carbonsäure.

Besonders bevorzugt sind diejenigen Verbindungen der Formel (I), in denen

X für Fluor,

Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatom je Alkylgruppe, Cyclopropylamino, Methoxyamino, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, $R^1$ für Ethyl, Cyclopropyl, 2-Hydroxyethyl, 4-Fluorphenyl oder 2,4-Difluorphenyl,

$R^2$ für Wasserstoff, Alkyl mit 1 bis 2 Kohlenstoffatomen

$R^3$ für eine cyclische Aminogruppe wie

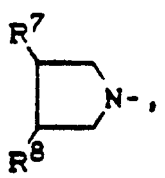 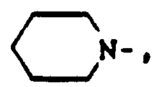 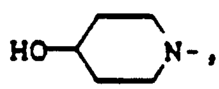 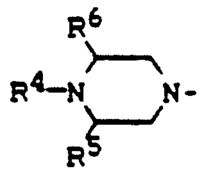

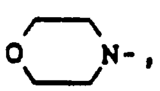 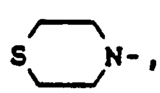 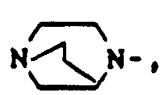 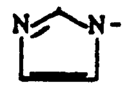 , 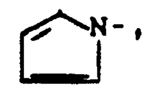

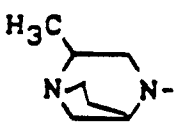

steht, worin

$R^4$ für Wasserstoff, Alkyl mit 1 bis 2 Kohlenstoffatomen, 2-Hydroxyethyl, 2-Oxopropyl, 3-Oxobutyl, Phenacyl, 4-Aminobenzyl,

$R^5$ für Wasserstoff oder Methyl,

$R^6$ für Wasserstoff, Methyl, Phenyl, 4-Fluorphenyl,

$R^7$ für Wasserstoff, Amino, Aminomethyl, Methylaminomethyl, Ethylaminomethyl, Hydroxy oder Hydroxymethyl,

$R^8$ für Wasserstoff steht,

A für N oder C-$R^9$ steht, worin

$R^9$ für Wasserstoff, Halogen wie Fluor oder Chlor steht oder auch gemeinsam mit R' eine Brücke der

Struktur
$-O-CH_2-\underset{|}{C}H-CH_3$

bilden kann,
und deren pharmazeutisch verwendbaren Hydrate und Säureadditionssalze sowie die Alkali-, Erdalkali-, Silber-und Guanidiniumsalze oder zugrundeliegenden Carbonsäuren ausgenommen 8-Amino-9-fluor-3-methyl-10-(4-methyl-1-piperazinyl)-7-oxo-2,3-dihydro-7H-pyrido[1,2,3-de][1,4]-benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl bedeutet und 5-Amino-7-(3-amino-1-pyrrolidinyl)-1-ethyl-6-fluor-1,4-dihydro4-oxo-1,8-naphthyridin-3-carbonsäure.

Weiterhin wurde gefunden, daß man die Verbindungen der Formel (I) erhält, wenn Verbindungen der Formel (II)

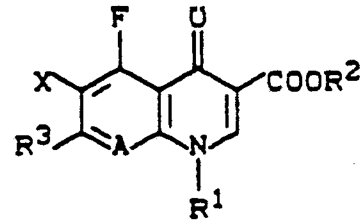

(II),

in welcher
X, R', $R^2$, $R^3$ und A die oben angegebene Bedeutung haben,
mit Verbindungen der Formel (III)

Y-H (III),

in welcher
Y die oben angegebene Bedeutung hat,
gegebenenfalls in Gegenwart von Säurefängern umsetzt.

Verwendet man beispielsweise 1-Cyclopropyl-5,6,8-trifluor-1,4-dihydro-7-(3-methyl-1-piperazinyl)-4-oxo-3-chinolincarbonsäure und Ammoniak als Ausgangsstoffe, so kann der Reaktionsablauf durch das folgende Formelschema wiedergegeben werden:

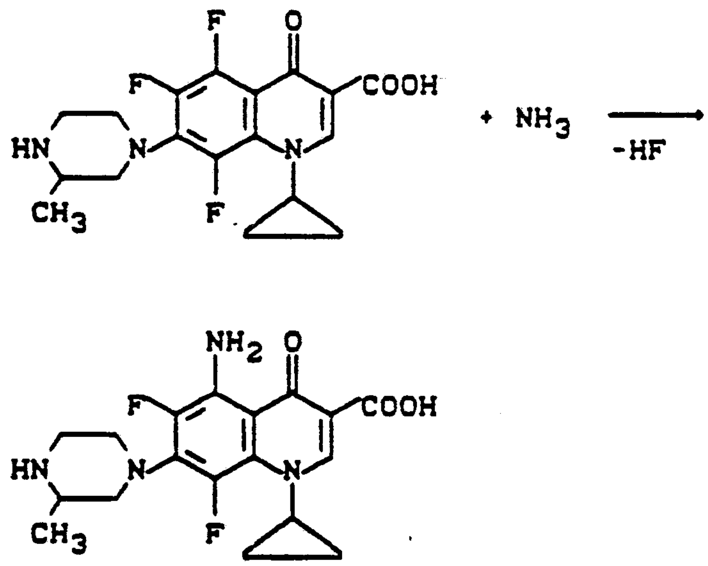


Die als Ausgangsstoffe verwendeten Verbindungen der Formel (II) sind bekannt oder können nach bekannten Methoden hergestellt werden (Europäische Patentanmeldung 202 763, Deutsche Patentanmeldung 35 22 405). Als Beispiele seien genannt:

7-[3-(Aminomethyl)-1-pyrrolidinyl]-1-cyclopropyl-5,6,8-trifluor-1,4-dihydro-4-oxo-3-chinolincarbonsäure,

1-Cyclopropyl-7-[3-[(methylamino]methyl]-1-pyrrolidinyl]-5,6,8-trifluor-1,4-dihydro-4-oxo-3-chinolincarbonsäure,
1-Cyclopropyl-7-[3-[(ethylamino)methyl]-1-pyrrolidinyl]-5,6,8-trifluor-1,4-dihydro-4-oxo-3-chinolincarbonsäure.
7-[3-(Amino-1-pyrrolidinyl]-1-cyclopropyl-5,6,8-trifluor-1,4-dihydro-4-oxo-3-chinolincarbonsäure,
1-Cyclopropyl-7-[3-[(ethylamino)methyl]-1-pyrrolidinyl]-5,6,8-trifluor-1,4-dihydro-4-oxo-3-chinolincarbonsäure,
1-Cyclopropyl-5,6,8-trifluor-1,4-dihydro-7-[3-[[(1-methylethyl)amino]methyl]-1-pyrrolidinyl]-4-oxo-3-chinolincarbonsäure,
7-[3-(Aminomethyl)-1-pyrrolidinyl]-1-cyclopropyl-1,4-dihydro-5,6 -difluor-4-oxo-1,8-naphthyridin-3-carbonsäure,
1-Cyclopropyl-7-[3-[(ethylamino)methyl]-1-pyrrolidinyl]-1,4-dihydro-5,6-difluor-4-oxo-1,8-naphthyridin-3-carbonsäure,
7-[3-Amino-1-pyrrolidinyl]-1-cyclopropyl-1,4-dihydro-5,6-difluor-4-oxo-1,8-naphthyridin-3-carbonsäure.
7-[3-(Aminomethyl)-1-pyrrolidinyl]-1-ethyl-5,6-difluor-1,4-dihydro-4-oxo-1,8-naphthyridin-3-carbonsäure,
7-[3-(Aminomethyl)-1-pyrrolidinyl]-1-ethyl-5,6,8-trifluor-1,4-dihydro-4-oxo-3-chinolincarbonsäure,
7-[3-(Aminomethyl)-1-pyrrolidinyl]-5,6,8-trifluor-1-(2-fluoroethyl)-1,4-dihydro-4-oxo-3-chinolincarbonsäure,
7-[3-(Aminomethyl)-1-pyrrolidinyl]-1-ethyl-5,6,8-trifluor-1-ethenyl-1,4-dihydro-4-oxo-3-chinolincarbonsäure.
1-Ethyl-7-[3-[(ethylamino)methyl]-1-pyrrolidinyl]-5,6-difluor-1,4-dihydro-4-oxo-1,8-naphthyridin-3-carbonsäure,
1-Ethyl-7-[3-[(ethylamino)methyl]-1-pyrrolidinyl]-5,6,8-trifluor-1,4-dihydro-4-oxo-3-chinolincarbonsäure,
7-[3-[(Ethylamino)methyl]-1-pyrrolidinyl]-5,6,8-trifluor-1-(2-fluorethyl)-1,4-dihydro-4-oxo-3-chinolincarbonsäure,
1-Ethenyl-7-[3-[(ethylamino)methyl-1-pyrrolidinyl]-5,6,8-trifluor-1,4-dihydro-4-oxo-3-chinolincarbonsäure,
1-Ethyl-5,6-difluor-1,4-dihydro-7-[3-[[(1-methylethyl)-amino]methyl]-1-pyrrolidinyl]-4-oxo-1,8-naphthyridin-3-carbonsäure,
1-Ethyl-7-[3-[(1-methylethyl)aminomethyl]-1-pyrrolidinyl]-5,6,8-trifluor-1,4-dihydro-4-oxo-3-chinolincarbonsäure.
1-Ethyl-5,6-difluor-1,4-dihydro-7-(7-methyl-2,7-diazaspiro[4,4]non-2-yl)-4-oxo-1,8-naphthyridin-3-carbonsäure,
1-Ethyl-5,6,8-trifluor-1,4-dihydro-7-(7-ethyl-2,7-diazaspiro[4,4]non-2-yl)-4-oxo-3-chinolincarbonsäure,
1-Cyclopropyl-5,6,8-trifluor-1,4-dihydro-7-(7-methyl-2,7-diazaspiro[4,4]non-2-yl]-4-oxo-3-chinolincarbonsäure,
7-(3-Amino-1-pyrrolidinyl)-1-ethyl-5,6,8-trifluor-1,4-dihydro-4-oxo-3-chinolincarbonsäure,
1-Cyclopropyl-5,6-difluor-1,4-dihydro-7-(3-hydroxy-1-pyrrolidinyl)-4-oxo-3-chinolincarbonsäure,
1-Cyclopropyl-5,6,8-trifluor-1,4-dihydro-7-(3-hydroxy-1-pyrrolidinyl)-4-oxo-3-chinolincarbonsäure,
1-Cyclopropyl-5,6-difluor-1,4-dihydro-7-(3-hydroxy-1-pyrrolidinyl)-4-oxo-3-chinolincarbonsäure,
1-Cyclopropyl-7-(1,4-diazabicyclo[3.2.1]oct-4-yl)-5,6,8-trifluor-1,4-dihydro-4-oxo-3-chinolincarbonsäure,
1-Cyclopropyl-7-(1,4-diazabicyclo[3.2.1]oct-4-yl)-5,6-difluor-1,4-dihydro-4-oxo-1,8-naphthyridin-3-carbonsäure,
1-Cyclopropyl-5,6,8-trifluor-1,4-dihydro-7-(2-methyl-1,4-diazabicyclo[3.2.1]oct-4-yl)-4-oxo-3-chinolincarbonsäure,
1-Cyclopropyl-5,6,8-trifluor-1,4-dihydro-7-(8-methyl-3,8-diazabicyclo[3.2.1]oct-3-yl)-4-oxo-3-chinolincarbonsäure,
1-Cyclopropyl-5,6,8-trifluor-1,4-dihydro-4-oxo-7-(1-piperazinyl)-3-chinolincarbonsäure,
1-Cyclopropyl-5,6,8-trifluor-1,4-dihydro-4-oxo-7-(4-methyl-1-piperazinyl)-3-chinolincarbonsäure,
1-Cyclopropyl-5,6,8-trifluor-1,4-dihydro-4-oxo-7-(4-ethyl-1-piperazinyl)-3-chinolincarbonsäure,
1-Cyclopropyl-5,6,8-trifluor-1,4-dihydro-7-[4-(2-hydroxyethyl)-1-piperazinyl)-4-oxo-3-chinolincarbonsäure,
1-Cyclopropyl-5,6,8-trifluor-1,4-dihydro-4-oxo-7-(3-methyl-1-piperazinyl)-3-chinolincarbonsäure,
1-Cyclopropyl-5,6,8-trifluor-1,4-dihydro-4-oxo-7-(3,5-dimethyl-1-piperazinyl)-3-chinolincarbonsäure,
1-Cyclopropyl-5,6,8-trifluor-1,4-dihydro-4-oxo-7-morpholino-3-chinolincarbonsäure,
1-Cyclopropyl-5,6,8-trifluor-1,4-dihydro-4-oxo-7-thiomorpholino-3-chinolincarbonsäure,
1-Cyclopropyl-5,6,8-trifluor-1,4-dihydro-4-oxo-7-(4-isopropyl-1-piperazinyl)-3-chinolincarbonsäure,
7-(4-Allyl-1-piperazinyl)-1-cyclopropyl-5,6,8-trifluor-1,4-dihydro-4-oxo-3-chinolincarbonsäure,
1-Cyclopropyl-5,6,8-trifluor-1,4-dihydro-4-oxo-7-[4-(2-oxopropyl)-1-piperazinyl]-3-chinolincarbonsäure,
1-Cyclopropyl-5,6,8-trifluor-1,4-dihydro-4-oxo-7-[4-(3-oxobutyl)-1-piperazinyl]-3-chinolincarbonsäure,
1-Cyclopropyl-5,6,8-trifluor-1,4-dihydro-4-oxo-7-(3-phenyl-1-piperazinyl)-3-chinolincarbonsäure,
1-Cyclopropyl-5,6,8-trifluor-1,4-dihydro-7-(1-imidazolyl)-4-oxo-3-chinolincarbonsäure,
1-Cyclopropyl-5,6,8-trifluor-1,4-dihydro-4-oxo-7-(1-pyrrolyl)-3-chinolincarbonsäure,
S-8,9-Difluor-3-methyl-10-(4-methyl-1-piperazinyl)-7-oxo-7H-pyrido[1,2,3-de][1,4]benzoxazin-6-carbonsäure.

Die als Ausgangsverbindungen verwendeten Verbindungen der Formel (III) sind bekannt. Als Beispiele

seien genannt: Ammoniak, Methylamin, Ethylamin, Propylamin, Isopropylamin, Butylamin, Dimethylamin, Ethyl-methylamin, Diethylamin, 2-Hydroxyethylamin, Ethylendiamin, 2-(tert.-Butoxycarbonylamino)-ethylamin, 2-Methoxyethylamin, Cyclopropylamin, Methoxyamin, Butoxyamin, Methanol, Methylmercaptan, Thiophenol, Mercaptoessigsäureethylester, Mercaptoessigsäure, Hydrazin.

Die Umsetzung von (II) mit (III) wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether, Acetonitril oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, Natriumhydrid, organische Amine und Amidine. Als besonders geeignet seien im einzelnen genannt: Triethylamin, 1,4-Diaza-bicyclo-[2.2.2]octan (DABCO), 1,8-Diazabicyclo[5.3.0]undec-7-en (DBU).

Die Reaktionstemperaturen können in einem großeren Bereicht varriert werden. Im allgemeinen arbeitet man zwischen etwa 70 und 200°C, vorzugsweise zwischen 100 und 180°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 50 Mol, vorzugsweise 1 bis 30 Mol der Verbindung (III) ein.

In einzelnen Fällen können auch Schutzgruppen verwendet werden, die man nach beendeter Umsetzung von (II) mit (III) nachträglich abspalten kann. So werden beispielsweise Ester durch Erhitzen in Gegenwart von Schwefelsäure auf 100 bis 150° hydrolytisch zu Carbonsäuren gespalten. Die Abspaltung des tert.-Butoxycarbonyl-Restes als Amino-Schutzgruppe erfolgt in Gegenwart von Säuren wie beispielsweise Salzsäure, Bromwasserstoffsäure (siehe Houben-Weyl, Methoden der organischen Chemie, Band E4, Seite 144 (1983)).

Unter den gleichen sauren Bedingungen erfolgt auch die Abspaltung des Tetrahydroxypyranylrestes als Hydroxy-Schutzgruppe (siehe J.F.W. McOmie, Protective Groups in Organic Chemistry (1973), Seite 104).

Zur Herstellung der erfindungsgemäßen Ester kann die zugrundeliegende Carbonsäure auch in überschüssigem Alkohol in Gegenwart von starken Säuren, wie Schwefelsäure, wasserfreiem Chlorwasserstoff, Methansulfonsäure, p-Toluolsulfonsäure oder sauren Ionenaustauschern, bei Temperaturen von etwa 20° bis 200°C, vorzugsweise etwa 60° bis 120°C umgesetzt werden. Das entstehende Reaktionswasser kann auch durch azeotrope Destillation mit Chloroform, Tetrachlormethan, Benzol oder Toluol entfernt werden.

Die als Prodrug verwendeten (5-Methyl-2-oxo-1,3-dioxol-4-yl-methyl)-ester können auch durch Umsetzung eines Alkalisalzes der zugrundeliegenden Carbonsäure mit 4-Brommethyl-oder 4-Chlormethyl-5-methyl-1,3-dioxol-2-on in einem Lösungsmittel wie Dimethylformamid, Dimethylacetamid, Dimethylsulfoxid oder Tetramethylharnstoff bei Temperaturen von etwa 0° bis 100°C, vorzugsweise 0° bis 50°C, erhalten werden.

Die Einführung eines Aminobenzylrestes $R^4$ kann auch durch Reduktion eines bereits im Wirkstoff der Formel I eingeführten Nitrobenzylrestes durch katalytisch angeregten Wasserstoff oder chemisch durch Reduktion mit Eisen oder Zink erfolgen.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonomethylether erhitzen und anschließend bis zur Trockne eindampfen oder das ausgefallene Salz absaugen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Embonsäure, Glutaminsäure oder Asparaginsäure zu verstehen.

Die Alkali-oder Erdalkalisalze der erfindungsgemäßen Carbonsäuren werden beispielsweise durch Lösen des Betains in unterschüssiger Alkali-oder Erdalkalilauge, Filtration von ungelöstem Betain und Eindampfen des Filtrats bis zur Trockne erhalten. Pharmazeutisch geeignet sind Natrium-, Kalium-oder Calciumsalze. Durch Umsetzung eines Alkali-oder Erdalkalisalzes mit einem geeigneten Silbersalz wie Silbernitrat werden die entsprechenden Silbersalze erhalten.

Die erfindungsgemäßen Wirkstoffe mit einen asymmetrischen Kohlenstoffatom im Rest $R^3$ können sowohl als Racemate als auch als enantiomerenreine Verbindungen vorliegen.

Außer den in den Beispielen aufgeführten Verbindungen seien als neue Wirkstoffe im einzelnen genannt:

5-Amino-7-[3-(aminomethyl)-1-pyrrolidinyl]-1-cyclopropyl-6,8-difluor-1,4-dihydro-4-oxo-3-chinolincarbonsäure,

5-Amino-1-cyclopropyl-7-[3-[(methylamino)methyl]-1-pyrrolidinyl]6,8-difluor-1,4-dihydro-4-oxo-3-chinolincarbonsäure,

5-Amino-1-cyclopropyl-7-[3-[(ethylamino)methyl]-1-pyrrolidinyl]-6,8-difluor-1,4-dihydro-4-oxo-3-chinolincarbonsäure,

5-Amino-7-[3-amino-1-pyrrolidinyl]-1-cyclopropyl-6,8-difluor-1,4-dihydro-4-oxo-3-chinolincarbonsäure,

5-Amino-1-cyclopropyl-7-[3-(ethylamino)-1-pyrrolidinyl]-6,8-difluor-1,4-dihydro-4-oxo-3-chinolincarbonsäure,

5-Amino-1-cyclopropyl-6,8-difluor-1,4-dihydro-7-[3-[[(1-methylethyl)amino]methyl]-1-pyrrolidinyl]-4-oxo-3-chinolincarbonsäure,

5-Amino-7-[3-(aminomethyl)-1-pyrrolidinyl]-1-cyclopropyl-1,4-dihydro-6-fluor-4-oxo-1,8-naphthyridin-3-carbonsäure,

5-Amino-1-cyclopropyl-7-[3-[(ethylamino)methyl]-1-pyrrolidinyl]-1,4-dihydro-6-fluor-4-oxo-1,8-naphthyridin-3-carbonsäure,

5-Amino-7-[3-amino-1-pyrrolidinyl]-1-cyclopropyl-1,4-dihydro-6-fluor-4-oxo-1,8-naphthyridin-3-carbonsäure,

5-Amino-7-[3-(aminomethyl)-1-pyrrolidinyl]-1-ethyl-6-fluor-1,4-dihydro-4-oxo-1,8-naphthyridin-3-carbonsäure,

5-Amino-7-[3-aminomethyl)-1-pyrrolidinyl]-1-ethyl-6,8-difluor-1,4-dihydro-4-oxo-3-chinolincarbonsäure,

5-Amino-7-[3-(aminomethyl)-1-pyrrolidinyl]-6,8-difluor-1-(2-fluoroethyl)-1,4-dihydro-4-oxo-3-chinolincarbonsäure,

5-Amino-7-[3-(aminomethyl)1-pyrrolidinyl]-6,8-difluor-1-ethenyl-1,4-dihydro-4-oxo-3-chinolincarbonsäure,

5-Amino-1-ethyl-7-[3-[(ethylamino)methyl]-1-pyrrolidinyl]-6-fluor-1,4-dihydro-4-oxo-1,8-naphthyridin-3-carbonsäure,

5-Amino-1-ethyl-7-[3-[(ethylamino)methyl]-1-pyrrolidinyl]-6,8-difluor-1,4-dihydro-4-oxo-3-chinolincarbonsäure,

5-Amino-7-[3-[(ethylamino)methyl]-1-pyrrolidinyl]-6,8-difluor-1-(2-fluorethyl)-1,4-dihydro-4-oxo-3-chinolincarbonsäure,

5-Amino-1-ethenyl-7-[3-[(ethylamino)methyl-1-pyrrolidinyl]-6,8-difluor-1,4-dihydro-4-oxo-3-chinolincarbonsäure,

5-Amino-1-ethyl-6-fluor-1,4-dihydro-7-[3-[[(1-methylethyl)-amino]methyl]-1-pyrrolidinyl]-4-oxo-1,8-naphthyridin-3-carbonsäure,

5-Amino-1-ethyl-7-[3-[(1-methylethyl)aminomethyl]-1-pyrrolidinyl]-6,8-difluor-1,4-dihydro-4-oxo-3-chinolincarbonsäure,

5-Amino-1-ethyl-6-fluor-1,4-dihydro-7-(7-methyl-2,7-diazaspiro[4.4]non-2-yl)-4-oxo-1,8-naphthyridin-3-carbonsäure,

5-Amino-1-ethyl-6,8-difluor-1,4-dihydro-7-(7-ethyl-2,7-diazaspiro[4.4]non-2-yl)-4-oxo-3-chinolincarbonsäure,

5-Amino-1-cyclopropyl-6,8-difluor-1,4-dihydro-7-(7-methyl-2,7-diazaspiro[4.4]non-2-yl]-4-oxo-3-chinolincarbonsäure,

5-Amino-7-(3-amino-1-pyrrolidinyl)-1-ethyl-6,8-difluor-1,4-dihydro-4-oxo-3-chinolincarbonsäure,

5-Amino-1-cyclopropyl-6-fluor-1,4-dihydro-7-(3-hydroxy-1-pyrrolidinyl)-4-oxo-3-chinolincarbonsäure,

5-Amino-1-cyclopropyl-6-fluor-1,4-dihydro-7-(3-hydroxy-1-pyrrolidinyl)-4-oxo-3-chinolincarbonsäure,

5-Amino-1-cyclopropyl-7-(1,4-diazabicyclo[3.2.1]oct-4-yl)-6-fluor-1,4-dihydro-4-oxo-1,8-naphthyridin-3-carbonsäure,

5-Amino-1-cyclopropyl-6,8-difluor-1,4-dihydro-7-(2-methyl-1,4-diazabicyclo[3.2.1]oct-4-yl)-4-oxo-3-chinolincarbonsäure,

5-Amino-1-cyclopropyl-6,8-difluor-1,4-dihydro-7-(8-methyl-3,8-diazabicyclo[3.2.1]oct-3-yl)-4-oxo-3-chinolincarbonsäure,

5-Amino-1-cyclopropyl-6,8-difluor-1,4-dihydro-4-oxo-7-(1-piperazinyl)-3-chinolincarbonsäure,

5-Amino-1-cyclopropyl-6,8-difluor-1,4-dihydro-4-oxo-7-thiomorpholino-3-chinolincarbonsäure,

5-Amino-1-cyclopropyl-6,8-difluor-1,4-dihydro-4-oxo-7-(4-isopropyl-1-piperazinyl)-3-chinolincarbonsäure,

5-Amino-7-(4-allyl-1-piperazinyl)-1-cyclopropyl-6,8-difluor-1,4-dihydro-4-oxo-3-chinolincarbonsäure,

5-Amino-1-cyclopropyl-6,8-difluor-1,4-dihydro-4-oxo-7-[4-(2-oxopropyl)-1-piperazinyl]-3-chinolincarbonsäure,

5-Amino-1-cyclopropyl-6,8-fluor-1,4-dihydro-4-oxo-7-[4-(3-oxobutyl)-1-piperazinyl]-3-chinolincarbonsäure,

5-Amino-1-cyclopropyl-6,8-fluor-1,4-dihydro-4-oxo-7-(1-pyrrolyl)-3-chinolincarbonsäure,

5-Amino-7-[4-(4-aminobenzyl)-1-piperazinyl]-1-cyclopropyl-6,8-difluor-1,4-dihydro-4-oxo-3-chinolincarbonsäure,

5-Amino-1-cyclopropyl-6,8-difluor-1,4-dihydro-4-oxo-7-(4-phenacyl-1-piperazinyl)-3-chinolincarbonsäure,

S-8-Amino-9-fluor-3-methyl-10-(4-methyl-1-piperazinyl)-7-oxo-7H-pyrido[1,2,3-de][1,4]benzoxazin-6-carbonsäure,

5-(2-Aminoethylamino)-1-cyclopropyl-6,8-difluor-1,4-dihydro-7-(4-methyl-1-piperazinyl)-4-oxo-3-chinolincarbonsäure.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan(IV)oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penecilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Propylaxe und Chemotherapie von lokalen und systemischen Infektionen in der Human-und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph, aureus, Staph. epidermidis) und Streptokokken (Strept. agalactiae, Strept. faecalis, Strept. pneumoniae, Strept. pyogenes); gram-negative Kokken (Neisseria gonoorhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs, oxytoca), Enterobacter (Ent. aerogenes, Ent. agglomerans), Hafnia, Serratia (Serr. marcescens),Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobacterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfin-

dungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen-und Gelenkinfektionen, zystische Fibrose, Hautinfektionen, postoperative, Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augen-infektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis:

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll-und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium-und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B.

$C_{1-4}$-Alkohol mit $C_{1-6}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lö sungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zum parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylakohole, Polyoxyethylensorbit-und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber-und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Taletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe (Kunststoffketten zur lokalen Therapie), Kollagen oder Knochenzement eingearbeitet werden.

Im allgemeinen hat es sich sowohl in der Human-als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden

und dadurch eine Förderung des Wachstums und eine Verbesserung der Verwertung des Futters erreicht werden.

**Tabelle**: Minimale Hemmkonzentrationen (mg/l) *

| Teststamm: | 21 | 23 | 24 | 25 | 26 | 27 | 28 | 32 | 30 |
|---|---|---|---|---|---|---|---|---|---|
| | Beispiele | | | | | | | | |
| **E.coli** Neumann | ≤0,015 | ≤0,015 | 0,125 | 0,125 | 0,03 | 0,125 | ≤0,015 | ≤0,015 | 0,03 |
| 455/7 | 2 | 1 | 4 | 1 | 16 | 16 | 2 | 1 | 16 |
| A261 | 0,5 | 0,25 | 1 | 0,125 | 4 | 2 | 0,25 | 0,06 | 2 |
| **Klebsiella** 63 | 0,06 | ≤0,015 | 0,125 | 0,03 | 0,06 | 0,125 | ≤0,015 | ≤0,015 | 0,06 |
| 8085 | 0,06 | 0,03 | 0,125 | 0,06 | 0,06 | 0,125 | ≤0,015 | 0,03 | 0,03 |
| **Proteus** mir. 8223 | 0,5 | 0,125 | 2 | 1 | 0,5 | 0,25 | 0,06 | 0,5 | 2 |
| 8175 | 0,125 | 0,06 | 0,25 | 0,125 | 0,125 | 0,25 | ≤0,015 | 0,06 | 0,06 |
| vulg. 1017 | ≤0,015 | ≤0,015 | 0,06 | ≤0,015 | 0,03 | 0,06 | ≤0,015 | ≤0,015 | ≤0,015 |
| **Morg.** morg. 932 | 0,03 | ≤0,015 | 0,125 | ≤0,015 | 0,03 | 0,06 | ≤0,015 | ≤0,015 | ≤0,015 |
| 11006 | 0,125 | 0,03 | 0,25 | 0,03 | 0,125 | 0,125 | ≤0,015 | 0,03 | 0,06 |
| **Providencia** 12012 | 0,06 | ≤0,015 | 0,125 | 0,06 | 0,06 | 0,06 | ≤0,015 | 0,03 | ≤0,015 |
| 12052 | 2 | 1 | 4 | 4 | 8 | 8 | 1 | 1 | 4 |
| **Serratia** 16040 | 4 | 2 | 8 | 4 | 8 | 16 | 1 | 4 | 8 |
| **Staph.** FK 422 | 0,06 | ≤0,015 | - | ≤0,015 | 0,03 | ≤0,015 | ≤0,015 | 0,03 | ≤0,015 |
| 1756 | 0,06 | ≤0,015 | 0,06 | ≤0,015 | 0,03 | ≤0,015 | ≤0,015 | 0,03 | ≤0,015 |
| 133 | 0,06 | ≤0,015 | 0,06 | ≤0,015 | 0,03 | ≤0,015 | ≤0,015 | 0,03 | 0,03 |
| **Enteroc.** 27101 | 0,25 | 0,03 | 0,25 | 0,125 | 0,25 | 0,03 | 0,03 | 0,03 | 0,25 |
| 9790 | 0,25 | 0,06 | 0,25 | 0,125 | 0,25 | 0,125 | 0,03 | 0,03 | 0,25 |
| **Pseudomonas** Walter | 1 | 1 | 2 | 0,5 | 2 | 1 | 0,25 | 1 | 2 |
| Ellsworth | ≤0,015 | ≤0,015 | 0,25 | 0,03 | 0,03 | 0,125 | 0,03 | 0,03 | 0,25 |

*) Die MHK-Werte wurden im Agarverdünnungstest gemessen. Agarplatten mit Isosensitest-Agar (Oxoid) wurden mit einem Multipoint-Inokulator beimpft. Das Inokulum betrug ca. $10^4$ Keime pro Impfpunkt.

**Tabelle (Fortsetzung)**

| Teststamm: | Beispiele 19 | 20 | 17 | 22 |
|---|---|---|---|---|
| E.coli Neumann | 0,03 | ≤0,015 | ≤0,015 | ≤0,015 |
| 455/7 | 1 | 2 | 0,5 | 0,5 |
| A261 | 0,25 | 0,25 | 0,125 | 0,125 |
| Klebsiella 63 | 0,03 | 0,03 | ≤0,015 | 0,03 |
| 8085 | 0,03 | 0,06 | ≤0,015 | 0,06 |
| Proteus mir. 8223 | 0,25 | 0,25 | 0,25 | 0,50 |
| 8175 | 0,125 | 0,125 | 0,06 | 0,06 |
| vulg. 1017 | 0,06 | ≤0,015 | ≤0,015 | ≤0,015 |
| Morg. morg. 932 | 0,03 | 0,03 | ≤0,015 | 0,03 |
| 11006 | 0,06 | 0,06 | ≤0,015 | 0,03 |
| Providencia 12012 | 0,06 | 0,06 | ≤0,015 | 0,03 |
| 12052 | 1 | 1 | 2 | 1 |
| Serratia 16040 | 4 | 4 | 2 | 2 |
| Staph. FK 422 | 0,03 | 0,06 | 0,03 | 0,03 |
| 1756 | 0,06 | 0,06 | 0,03 | 0,03 |
| 133 | 0,06 | 0,06 | 0,03 | 0,03 |
| Enteroc. 27101 | 0,25 | 0,25 | 0,125 | 0,125 |
| 9790 | 0,25 | 0,25 | 0,125 | 0,25 |
| Pseudomonas Walter | 0,5 | 1 | 0,25 | 0,5 |
| Ellsworth | 0,06 | 0,03 | ≤0,015 | 0,03 |

Beispiel A

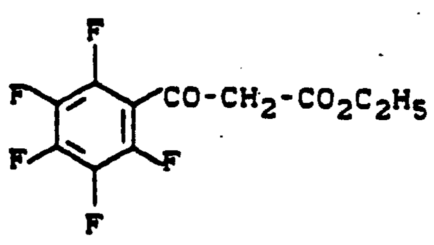

11,1 g Magnesiumspäne werden in 25 ml wasserfreiem Ethanol suspendiert. Man versetzt mit 2,5 g Tetrachlorkohlenstoff und tropft, wenn die Reaktion in Gang gekommen ist, ein Gemisch von 79 g Malonsäurediethylester, 50 ml abs. Ethanol und 200 ml wasserfreiem Diethylether unter Rückfluß zu. Dann wird noch 2 Stunden auf Rückflußtemperatur erhitzt, mit Ein/Methanol auf 0°C gekühlt und bei dieser Temperatur eine Lösung von 106,3 g (0,5 mol) Pentafluorbenzoylfluorid in 135 ml Diethylether langsam

zugetropft. Man rührt 1 Stunde bei 0°C, läßt über Nacht auf Raumtemperatur kommen und läßt unter Eiskühlung ein Gemisch von 200 ml Eiswasser und 12,4 ml konzentrierter Schwefelsäure zulaufen. Die Phasen werden getrennt und zweimal mit Ether nachextrahiert. Die vereinigten Etherlösungen werden mit Wasser gewaschen, mit $Na_2SO_4$ getrocknet und das Lösungsmittel im Vakuum abgezogen. Man erhält 170,8 g Pentafluorbenzoyl-malonsäurediethylester als Rohprodukt.

Eine Emulsion von 170 g rohem Pentafluorbenzoyl-malonsäurediethylester in 335 ml Wasser wird mit 0,7 g p-Toluolsulfonsäure versetzt. Man erhitzt unter gutem Rühren 3,5 Stunden zum Sieden, extrahiert die erkaltete Emulsion mehrmals mit Methylenchlorid, wäscht die vereinigten $CH_2Cl_2$-Lösungen einmal mit Wasser, trocknet mit $Na_2SO_4$ und destilliert das Lösungsmittel im Vakuum ab. Die Fraktionierung des Rückstandes (125,3 g) im Vakuum liefert 79,5 g (56 % der Theorie) Pentafluorbenzoylessigsäureethylester von Siedepunkt 77-79°C/0,13 mbar; $n_D^{20}$ : 1,4608.

Mit Pentafluorbenzoylchlorid verläuft die Umsetzung entsprechend.

Beispiel B

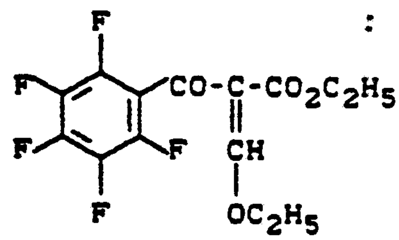

246 g (0,87 mol) Pentafluorbenzoyl-essigsäureethylester werden mit 189 g (1,25 mol) Orthoameisensäuretriethylester und 214 g (2,1 mol) Essigsäureanhydrid 2 Stunden unter Rückfluß erhitzt. Anschließend wird bis 140°C Badtemperatur im Vakuum eingeengt und 196 g (67 % der Theorie) 3-Ethoxy-2-(pentafluorbenzoyl)-acrylsäureethylester als Öl erhalten.

Beispiel C

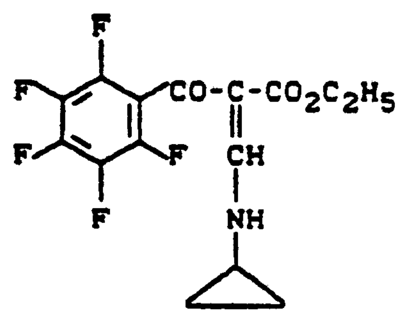

196 g (0,58 mol) 3-Ethoxy-2-(pentafluorbenzoyl)-acrylsäureethylester werden in 750 ml Ethanol gelöst und unter Kühlung 34,2 g (0,6 mol) Cyclopropylamin zugetropft. Man läßt 2 Stunden nachrühren, läßt über Nacht stehen, saugt das ausgefallene Kristallisat ab, wäscht mit kaltem Ethanol nach und trocknet (123,2 g, Schmelzpunkt 87-88° ). Die Mutterlauge wird auf die Hälfte eingeengt und eine weitere Fraktion (37,4 g, Schmelzpunkt 87-88° ) isoliert. Gesamtausbeute: 160,6 g (79 % der Theorie) 3-Cyclopropylamino-2-(pentafluorbenzoyl)-acrylsäureethylester.

Beispiel D

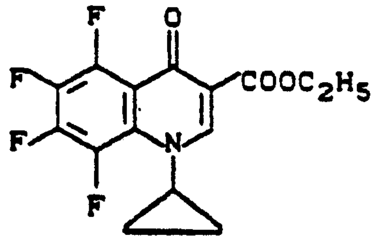

160,6 g (0,46 mol) 3-Cyclopropylamino-2-(pentafluorbenzoyl)-acrylsäureethylester werden in 700 ml Dimethylformamid mit 33,4 g (0,8 mol) Natriumfluorid versetzt und 3 Stunden unter Rückfluß erhitzt. Die Mischung wird auf 3 l Eiswasser gegossen, der Niederschlag abgesaugt, mit Wasser gewaschen und getrocknet.

Ausbeute: 144,6 g (96 % der Theorie) 1-Cyclopropyl-5,6,7,8-tetrafluor-1,4-dihydro-4-oxo-3-chinolincarbonsäureethylester vom Schmelzpunkt 169-173°.

Beispiel E

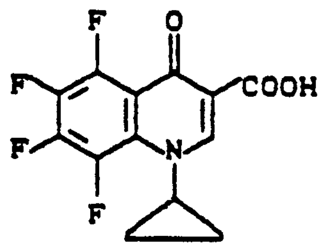

144,6 g (0,44 ml) 1-Cyclopropyl-5,6,7,8-tetrafluor-1,4-dihydro-4-oxo-3-chinolincarbonsäureethylester werden in einer Mischung aus 854 ml Essigsäure, 612 ml Wasser und 97 ml konzentrierter Schwefelsäure 3 Stunden unter Rückfluß erhitzt. Nach dem Abkühlen wird auf 3 l Eiswasser gegossen, der Niederschlag abgesaugt, mit Wasser gewaschen und getrocknet.

Ausbeute: 117,9 g (89 % der Theorie) 1-Cyclopropyl-5,6,7,8-tetrafluor-1,4-dihydro-4-oxo-3-chinolincarbonsäure vom Schmelzpunkt 176-178°; nach Umkristallisation aus Ethanol: Schmelzpunkt 178-180°.

Beispiel F

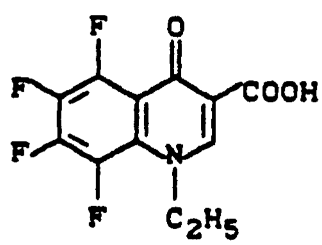

Man setzt 3-Ethoxy-2-(pentafluorbenzoyl)-acrylsäureethylester analog Beispiel C mit 50 %iger wäßriger Ethylamin-Lösung um und erhält 3-Ethylamino-2-(pentafluorbenzoyl)-acrylsäureethylester (cis-trans-Gemisch) vom Schmelzpunkt 87-88°, der analog Beispiel D mit Natriumfluorid zu 1-Ethyl-5,6,7,8-tetrafluor-1,4-dihydro-4-oxo-3-chinolincarbonsäureethylester vom Schmelzpunkt 216-221° (unter Zersetzung) cyclisiert wird. Dieser wird analog Beispiel E zu 1-Ethyl-5,6,7,8-tetrafluor-1,4-dihydro-4-oxo-3-chinolincarbonsäure

vom Schmelzpunkt 223-225° (unter Zersetzung) hydrolisiert.

Beispiel G

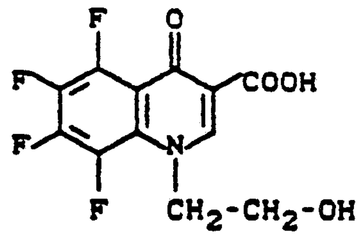

Führt man die in Beispiel F beschriebene Reaktionsfolge analog mit 2-Aminoethanol durch, dann erhält man über 3-(2-Hydroxyethylamino)-2-(pentafluorbenzoyl)-acrylsäureethylester (Öl, das sehr langsam kristallisiert; $R_f$-Wert: 0,7 [Laufmittel: Dichlormethan/Methanol/17 % wäßrige Ammoniaklösung 150:120:1; Kieselgel]) und 5,6,7,8-Tetrafluor-1,4-dihydro-1-(2-hydroxyethyl)-4-oxo-3-chinolincarbonsäureethylester (Schmelzpunkt: 200-203° unter Zersetzung) die 5,6,7,8-Tetrafluor-1,4-dihydro-1-(2-hydroxyethyl)-4-oxo-3-chinolincarbonsäure vom Schmelzpunkt 192-194° (unter Zersetzung).

Beispiel H

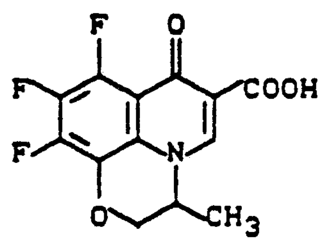

10,1 g (0,032 mol) 3-Ethoxy-2-(pentafluorbenzoyl)-acrylsäureethylester werden in 12 ml Ethanol vorgelegt. Unter Eiskühlung wird eine Lösung von 2,5 g (0,033 mol) 2-Amino-1-propanol in 12 ml Ethanol zugetropft. Man läßt anschließend zwei Stunden bei Raumtemperatur rühren und engt dann im Vakuum ein. Es werden 11,0 g 2-(Pentafluorbenzoyl)-3-(1-hydroxy-2-propylamino)-acrylsäureethylester als Rohprodukt (Öl) erhalten.

Dieses Rohprodukt wird mit 5,8 g Kaliumcarbonat in 50 ml Dimethylformamid vier Stunden auf 140° erhitzt. Nach Abkühlen auf Raumtemperatur wird mit Wasser versetzt. Der Niederschlag wird abgesaugt, getrocknet, mit Acetonitril verrührt und aus Glykolmonomethyletheracetat umkristallisiert.

Ausbeute: 2,4 g (24,3 %) 8,9,10-Trifluor-3-methyl-7-oxo-7H-pyrido[1,2,3-de][1,4]benzoxazin-6-carbonsäureethylester vom Schmelzpunkt 251-2° (unter Zersetzung).

2,0 g dieses Esters werden zusammen mit 7 ml Essigsäure, 5 ml Wasser und 0,6 ml Schwefelsäure vier Stunden auf 140° erhitzt. Nach Abkühlung versetzt man mit Wasser, isoliert den Feststoff und trocknet ihn.

Ausbeute: 1,5 g (91 %) 8,9,10-Trifluor-3-methyl-7-oxo-7H-pyrido[1,2,3-de][1,4]benzoxazin-6-carbonsäure vom Schmelzpunkt 300° (unter Zersetzung).

Die Umkristallisation aus Dimethylformamid verändert den Zersetzungspunkt nicht.

Beispiel 1

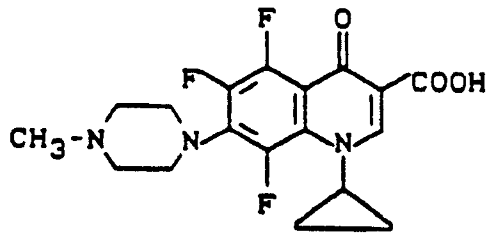

30 g (0.1 mol) 1-Cyclopropyl-5,6,7,8-tetrafluor-1,4-dihydro-4-oxo-3-chinolincarbonsäure werden in einer Mischung von 200 ml Acetonitril und 100 ml Dimethylformamid mit 15 g (0,15 mol) N-Methylpiperazin und 16,5 g (0,165 mol) 1,4-Diazabicyclo[2.2.2]octan versetzt und 3 Stunden unter Rückfluß erhitzt. Die Suspension wird eingeengt, der Rückstand mit Wasser verrührt, der ungelöste Niederschlag abgesaugt, mit Wasser und Methanol gewaschen und bei 80°C/12 mbar getrocknet.

Ausbeute: 20,3 g (53,5 % der Theorie) 1-Cyclopropyl-5,6,8-trifluor-1,4-dihydro-7-(4-methyl-1-piperazinyl)-4-oxo-3-chinolincarbonsäure vom Schmelzpunkt 210-220°C (unter Zersetzung); nach Umkristallisation aus Glykolmonomethylether: Schmelzpunkt 239-242° (unter Zersetzung).

Analog Beispiel 1 werden die Verbindungen der Beispiele 2 bis 14 erhalten:

Beispiel 2

1-Cyclopropyl-7-(4-ethyl-1-piperazinyl)-5,6,8-trifluor-1,4-dihydro-4-oxo-3-chinolincarbonsäure vom Schmelzpunkt 220-222° (unter Zersetzung) (aus Glykolmonomethylether).

Beispiel 3

1-Cyclopropyl-5,6,8-trifluor-1,4-dihydro-7-[4-(2-hydroxyethyl)-1-piperazinyl]-4-oxo-3-chinolincarbonsäure von Schmelzpunkt 244-247° (unter Zersetzung) (aus Glykolmonomethylether).

Beispiel 4

1-Cyclopropyl-5,6,8-trifluor-1,4-dihydro-7-(3-methyl-1-piperazinyl)-4-oxo-3-chinolincarbonsäure von Schmelzpunkt 235-236° (unter Zersetzung) (aus Glykolmonomethylether).

Beispiel 5

1-Cyclopropyl-5,6,8-trifluor-1,4-dihydro-7-(cis-3,5-dimethyl-1-piperazinyl)-4-oxo-3-chinolincarbonsäure vom Schmelzpunkt 257-258° (unter Zersetzung) (aus Glykolmonomethylether).

Beispiel 6

1-Cyclopropyl-5,6,8-trifluor-1,4-dihydro-4-oxo-7-(1-piperazinyl)-3-chinolincarbonsäure.

Beispiel 7

1-Cyclopropyl-5,6,8-trifluor-1,4-dihydro-4-oxo-7-(3-phenyl-1-piperazinyl)-3-chinolincarbonsäure vom Schmelzpunkt 198-199° (unter Zersetzung) (nach Reinigung durch Flashchromatographie an Kieselgel mit Dichlormethan/Methanol/17 % Ammoniak [30:8:1] als Laufmittel; $R_f$-Wert: 0,7).

Beispiel 8

1-Cyclopropyl-5.6,8-trifluor-7-[3-(4-fluorphenyl)-1-piperazinyl]-1,4-dihydro-4-oxo-3-chinolincarbonsäure vom Schmelzpunkt 205-206° (unter Zersetzung) (aus Glykolmonomethylether).

Beispiel 9

1-Cyclopropyl-7-(1,4-diazabicyclo[3.2.1]oct-4-yl)-5,6,8-trifluor-1,4-dihydro-4-oxo-3-chinolincarbonsäure vom Schmelzpunkt 319-321° (unter Zersetzung).

Beispiel 10

1-Cyclopropyl-5,6,8-trifluor-1,4-dihydro-7-morpholino-4-oxo-3-chinolincarbonsäure vom Schmelzpunkt 296-300° (unter Zersetzung) (Reinigung durch Verrühren mit Dichlormethan/Methanol/20 % Ammoniak [2:4:1]).

Beispiel 11

1-Cyclopropyl-5,6,8-trifluor-1,4-dihydro-4-oxo-7-(1-pyrrolidinyl)-3-chinolincarbonsäure vom Schmelzpunkt 314-316° (unter Zersetzung) (aus Dimethylformamid).

Beispiel 12

1-Cyclopropyl-5,6,8-trifluor-1,4-dihydro-7-(3-hydroxy-1-pyrrolidinyl)-4-oxo-3-chinolincarbonsäure vom Schmelzpunkt 281-282° (unter Zersetzung) (aus Glykolmonomethylether).

Beispiel 13

1-Cyclopropyl-7-(3-ethylaminomethyl-1-pyrrolidinyl)-5,6,8-trifluor-1,4-dihydro-4-oxo-3-chinolincarbonsäure vom Schmelzpunkt ab etwa 260° (unter Zersetzung).

Beispiel 14

1-Cyclopropyl-5,6,8-trifluor-1,4-dihydro-7-(1-imidazolyl)-4-oxo-3-chinolincarbonsäure vom Schmelzpunkt 207-208° (unter Zersetzung) (aus Glykolmonomethylether).

Beispiel 15

1,6 g (4,2 mmol) der Verbindung aus Beispiel 4 werden in 5 ml halbkonzentrierter Salzsäure gelöst, die Lösung filtriert und das Filtrat mit Ethanol bis zur Ausfällung des Hydrochlorids versetzt. Das Salz wird abgesaugt, mit Ethanol gewaschen und bei 100°/12 mbar getrocknet. Ausbeute: 1,1 g (63 % der Theorie) 1-Cyclopropyl-5,6,8-trifluor-1,4-dihydro-7-(3-methyl-1-piperazinyl)-4-oxo-3-chinolincarbonsäure-Hydrochlorid vom Schmelzpunkt 295° (unter Zersetzung).

Beispiel 16

Analog Beispiel 15 wird die Verbindung aus Beispiel 13 in 1-Cyclopropyl-7-(3-ethylaminomethyl-1-pyrrolidinyl)-5,6,8-trifluor-1,4-dihydro-4-oxo-3-chinolincarbonsäure-Hydrochlorid vom Schmelzpunkt 298° (unter Zersetzung) überführt.

Beispiel 17

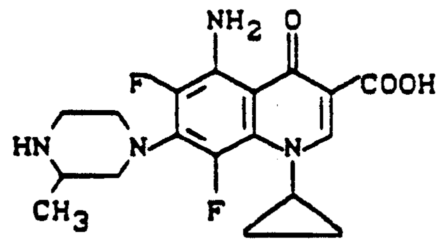

13,2 g (34,6 mmol) 1-Cyclopropyl-5,6,8-trifluor-1,4-dihydro-7-(3-methyl-1-piperazinyl)-4-oxo-3-chinolincarbonsäure werden in 112 ml Pyridin mit 56 ml gesättigter ethanolischer Ammoniaklösung versetzt und 8 Stunden bei 120° im Autoklaven erhitzt. Nach dem Abkühlen wird das aufgefallene gelbe Kristallisat abgesaugt, mit Wasser und Ethanol gewaschen und getrocknet.

Ausbeute: 9,3 g (71 % der Theorie) 5-Amino-1-cyclopropyl-6,8-difluor-1,4-dihydro-7-(3-methyl-1-piperazinyl)-4-oxo-3-chinolincarbonsäure vom Schmelzpunkt 231-232° (unter Zersetzung), nach Umkristallisation aus Glykolmonomethylether: Schmelzpunkt 239-242° (unter Zersetzung).

Führt man die Umsetzung während 12 Stunden bei 155° im Autoklaven durch, dann isoliert man das Reaktionsprodukt vom Schmelzpunkt 231-233° (unter Zersetzung) in 32 % Ausbeute.

Massenspektrum: m/e 378 ($M^+$), 358, 343, 322 (100 %, $M-C_3H_6N$), 278, 235, 180, 129, 70, 56, 41.

Beispiel 18 a

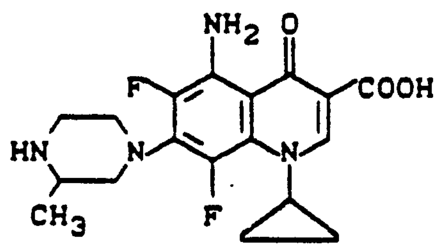

x $CH_3SO_3H$

300 mg (0,8 mmol) der Verbindung aus Beispiel 17 werden bei 70° in 7 ml Ethanol suspendiert und mit 0,1 g Methansulfonsäure versetzt. Man rührt ohne Heizung nach und läßt über Nacht abkühlen. Der Niederschlag wird abgesaugt, mit Ethanol gewaschen und bei 80°/0,1 mbar getrocknet.

Ausbeute: 260 mg (68,6 % der Theorie) 5-Amino-1-cyclopropyl-6,8-difluor-1,4-dihydro-7-(3-methyl-1-piperazinyl)-4-oxo-3-chinolincarbonsäure-Methansulfonat vom Schmelzpunkt ab 290° (unter Zersetzung).

$C_{18}H_{20}F_2N_4O_3$ x $CH_3SO_3H$ (474)

berechnet:

C 48,1 H 5,1 N 11,8 S 6,6

gefunden:

C 47,7 H 5,1 N 11,8 S 6,6

Beispiel 18 b

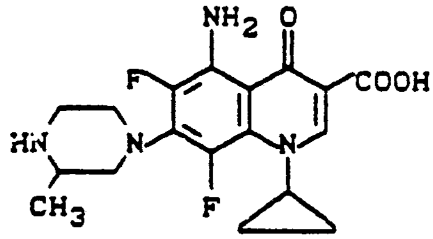

x HCl

29,5 g (78 mmol) der Verbindung aus Beispiel 17 werden in etwa 700 ml halbkonzentrierter Salzsäure heiß gelöst und filtriert. Das Filtrat wird abgekühlt, das ausgefallene Kristallisat abgesaugt, mit Ethanol gewaschen und getrocknet.
Ausbeute: 26,9 g (83 % der Theorie) 5-Amino-1-cyclopropyl-6,8-difluor-1,4-dihydro-7-(3-methyl-1-piperazinyl)-4-oxo-3-chinolincarbonsäure-hydrocylorid vom Schmelzpunkt ab 330° (unter Zersetzung).

Analog Beispiel 17 werden die Verbindungen der Beispiele 19 bis 29 erhalten.

Beispiel 19

5-Amino-1-cyclopropyl-6,8-difluor-1,4-dihydro-7-(4-methyl-1-piperazinyl)-4-oxo-3-chinolincarbonsäure vom Schmelzpunkt 239-241° (unter Zersetzung) (aus Glykolmonomethylether).

Beispiel 20

5-Amino-1-cyclopropyl-7-(4-ethyl-1-piperazinyl)-6,8-difluor-1,4-dihydro-4-oxo-3-chinolincarbonsäure vom Schmelzpunkt 220-222° (unter Zersetzung) (aus Glykolmonomethylether).

Beispiel 21

5-Amino-1-cyclopropyl-6,8-difluor-1,4-dihydro-7-[4-(2-hydroxyethyl)-4-oxo-3-chinolincarbonsäure vom Schmelzpunkt 244-245° (unter Zersetzung) (aus Glykolmonomethylether).

Beispiel 22

5-Amino-1-cyclopropyl-6,8-difluor-1,4-dihydro-7-(cis-3,5-dimethyl-1-piperazinyl)-4-oxo-3-chinolincarbonsäure vom Schmelzpunkt 250-253° (unter Zersetzung) (aus Glykolmonomethylether).

Beispiel 23

5-Amino-1-cyclopropyl-6,8-difluor-1,4-dihydro-4-oxo-7-(3-phenyl-1-piperazinyl)-3-chinolincarbonsäure vom Schmelzpunkt 242-243° (unter Zersetzung) (aus Glykolmonomethylether).

Beispiel 24

5-Amino-1-cyclopropyl-6,8-difluor-7-[3-(4-fluorphenyl)-1-piperazinyl]-1,4-dihydro-4-oxo-3-chinolincarbonsäure vom Schmelzpunkt 252-255° (unter Zersetzung) (aus Glykolmonomethylether).

Beispiel 25

5-Amino-1-cyclopropyl-7-(1,4-diazabicyclo[3.2.1]oct-4-yl)-6,8-difluor-1,4-dihydro-4-oxo-3-chinolincarbonsäure vom Schmelzpunkt 282-285° (unter Zersetzung).

Beispiel 26

5-Amino-1-cyclopropyl-6,8-difluor-1,4-dihydro-7-morpholino-4-oxo-3-chinolincarbonsäure vom Schmelzpunkt 287-290° (unter Zersetzung) (aus Glykolmonomethylether).

Beispiel 27

5-Amino-1-cyclopropyl-6,8-difluor-1,4-dihydro-4-oxo-7-(1-pyrrolidinyl)-3-chinolincarbonsäure vom Schmelzpunkt 241-244° (unter Zersetzung) (aus Glykolmonomethylether).

Beispiel 28

5-Amino-1-cyclopropyl-6,8-difluor-1,4-dihydro-7-(3-hydroxy-1-pyrrolidinyl)-4-oxo-3-chinolincarbonsäure vom Schmelzpunkt 258-259° (unter Zersetzung) (aus Glykolmonomethylether).

Beispiel 29

5-Amino-1-cyclopropyl-6,8-difluor-1,4-dihydro-7-(1-imidazolyl)-4-oxo-3-chinolincarbonsäure vom Schmelzpunkt 268-270° (unter Zersetzung) (aus Glykolmonomethylether).

Beispiel 30

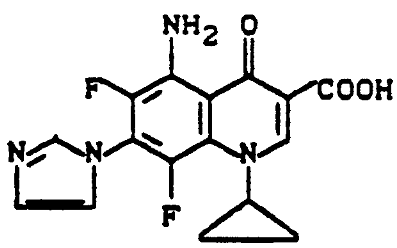 x $CH_3SO_3H$

0,9 g (2,6 mmol) der Verbindung aus Beispiel 29 werden in 10 ml Wasser suspendiert und langsam mit 0,6 g (6,2 mmol) Methansulfonsäure versetzt. Nach Zugabe von etwa 5/6 der Menge ist das Produkt weitgehend gelöst, danach tritt spontane Kristallisation ein. Das kompakte Kristallisiert wird mit weiteren 5 ml Wasser versetzt und durch Erwärmen in Lösung gebracht. Man läßt langsam auskristallisieren, verrührt mit 10 ml Ethanol, saugt ab, wäscht mit Ethanol und trocknet bei 100°/0,1 mbar.

Ausbeute: 0,87 g (75,7 % der Theorie) 5-Amino-1-cyclopropyl-6,8-difluor-1,4-dihydro-7-(1-imidazolyl)-4-oxo-3-chinolincarbonsäure-methansulfonat vom Schmelzpunkt 274-276° (unter Zersetzung).

Beispiel 31

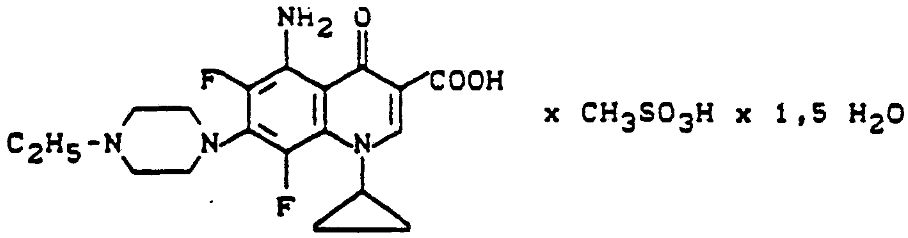

0,3 g (0,76 mmol) des Produktes aus Beispiel 20 werden in 7 ml Ethanol bei 70° suspendiert und mit 0,1 g Methansulfonsäure versetzt. Man rührt ohne Heizung nach und läßt 2 Tage stehen. Das Kristallisat wird abgesaugt und bei 80° 1 mbar getrocknet.
Ausbeute: 0,38 g (97 % der Theorie) 5-Amino-1-cyclopropyl-7-(4-ethyl-1-piperazinyl)-6,8-difluor-1,4-dihydro-4-oxo-3-chinolincarbonsäure-methansulfonat-hydrat vom Schmelzpunkt 271-273° (unter Zersetzung).

Beispiel 32

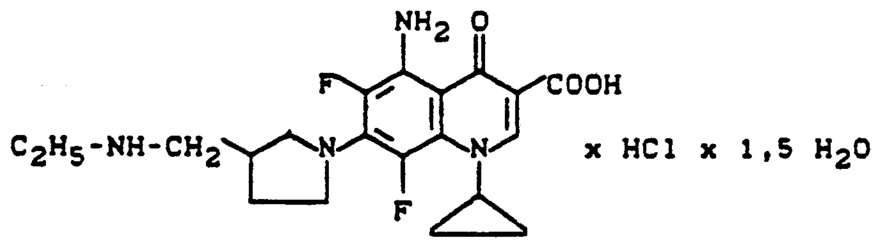

11,1 g (27 mmol) der Verbindung aus Beispiel 13 werden in 70 ml Pyridin mit 35 ml ethanolischer Ammoniaklösung versetzt und im Autoklaven 12 Stunden auf 155° erhitzt. Nach dem Abkühlen wird der ausgefallene Niederschlag abgesaugt, mit Wasser gewaschen und in 150 ml halbkonzentrierter Salzsäure gelöst. Die Lösung wird filtriert, eingeengt und der Rückstand mit Ethanol verrieben. Das ungelöste Hydrochlorid wird abgesaugt, mit Ethanol gewaschen und getrocknet.
Ausbeute: 5 g (39 % der Theorie) 5-Amino-1-cyclopropyl-7-(3-ethylaminomethyl-1-pyrrolidinyl)-6,8-difluor-1,4-dihydro-4-oxo-3-chinolincarbonsäure-hydrochlorid-hydrat vom Schmelzpunkt 225-229° (unter Zersetzung); nach Umkristallisation aus Glykolmonomethylether: Schmelzpunkt 245-250° (unter Zersetzung).

$C_{20}H_{24}F_2N_4O_3$ x HCl x 1,5 $H_2O$

berechnet:

C 51,1 H 5,9 N 11,9 Cl 7,6

gefunden:

C51,1 H 5,9 N 11,8 Cl 7,6

Beispiel 33

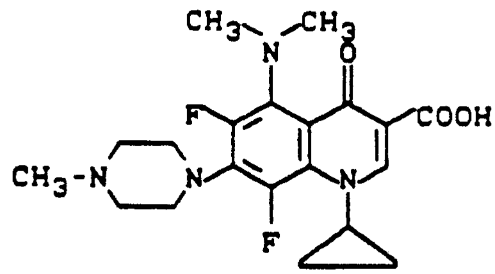

2.5 g (65.5 mmol) 1-Cyclopropyl-5,6,8-trifluor-1,4-dihydro-7-(4-methyl-1-piperazinyl)-4-oxo-3-chinolincarbonsäure werden in 15 ml Pyridin mit 7,5 ml gesättigter ethanolischer Dimethylaminlösung versetzt und im Autoklaven 6 Stunden auf 120° erhitzt. Nach dem Erkalten wird eingedampft, mit etwa 25 ml Wasser verrührt und das ausgefallene Produkt abgesaugt, mit Wasser gewaschen und getrocknet.
Ausbeute: 0,8 g (30 % der Theorie) 1-Cyclopropyl-5-dimethylamino-6-difluor-1,4-dihydro-7-(4-methyl-1-piperazinyl)-4-oxo-3-chinolincarbonsäure vom Schmelzpunkt 221-223° (unter Zersetzung).

Beispiel 34

Führt man die Umsetzung analog Beispiel 33 mit Methylamin durch, dann erhält man 1-Cyclopropyl-6,6-difluor-1,4-dihydro-5-methylamino-7-(4-methyl-1-piperazinyl)-4-oxo-3-chinolincarbonsäure vom Schmelzpunkt 226-229° (unter Zersetzung) (aus Glykolmonomethylether).

Beispiel 35

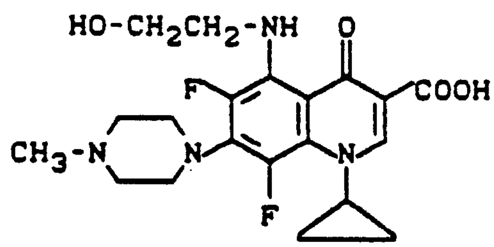

1,9 g (5 mmol) 1-Cyclopropyl-5,6,8-trifluor-1,4-dihydro-7-(4-methyl-1-piperazinyl)-4-oxo-3-chinolincarbonsäure werden in 20 ml Dimethylsulfoxid mit 0,6 g 1,4-Diazabicyclo[2.2.2]octan und 370 mg (5,7 mmol) 2-Aminoethanol versetzt und 2 Stunden auf 130 bis 140° erhitzt. Die Mischung wird eingedampft, der Rückstand mit 20 ml Wasser verrührt, der Niederschlag abgesaugt und aus Glykolmonomethylether umkristallisiert.
Ausbeute: 0,79 g (37,4 % der Theorie) 1-Cyclopropyl-6,8-difluor-1,4-dihydro-5-(2-hydroxyethylamino)-7-(4-methyl-1-piperazinyl)-4-oxo-3-chinolincarbonsäure vom Schmelzpunkt 189-190°.

Beispiel 36

Setzt man analog Beispiel 35 Cyclopropylamin ein, dann erhält mann 1-Cyclopropyl-5-cyclopropylamino-6,8-difluor-1,4-dihydro-7-(4-methyl-1-piperazinyl)-4-oxo-3-chinolincarbonsäure vom Schmelzpunkt 202-204° (unter Zersetzung).

Beispiel 37

Setzt man analog Beispiel 35 als Ausgangsverbindung Butylamin ein, dann erhält man 5-Butylamino-1-cyclopropyl-6,8-difluor-1,4-dihydro-7-(4-methyl-1-piperazinyl)-4-oxo-3-chinolincarbonsäure vom Schmelzpunkt 148-149° (unter Zersetzung).

Beispiel 38

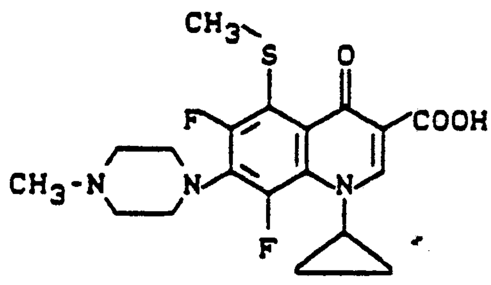

2,9 g (7,6 mmol) 1-Cyclopropyl-5,6,8-trifluor-1,4-dihydro-7-(4-methyl-1-piperazinyl)-4-oxo-3-chinolincarbonsäure werden in 30 ml Dimethylsulfoxid mit 1,7 g (15 mmol) 1,4-Diazabicyclo[2.2.2]octan versetzt und unter Eiskühlung etwa 450 mg Methylmercaptan einkondensiert. Man rührt ohne Kühlung nach und erhitzt 6 Stunden auf 110°. Nach dem Erkalten wird die Suspension in Wasser eingetragen, der Niederschlag abgesaugt, mit Wasser gewaschen und bei 80° 12 mbar getrocknet.
Ausbeute: 1,9 g (68 % der Theorie) 1-Cyclopropyl-6,8-difluor-1,4-dihydro-5-methylmercapto-7-(4-methyl-1-piperazinyl)-4-oxo-3-chinolincarbonsäure vom Schmelzpunkt 225-235° (unter Zersetzung); nach Umkristallisation aus Dimethylformamid: Schmelzpunkt 232-241° (unter Zersetzung).

Beispiel 39

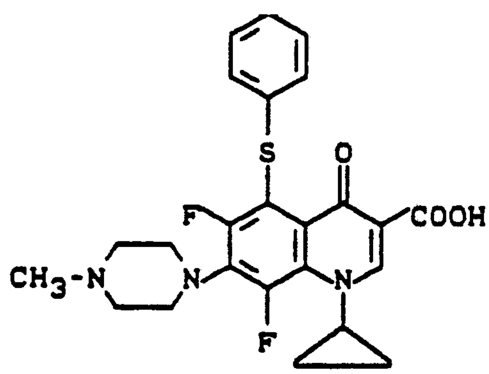

1,9 g (5 mmol) 1-Cyclopropyl-5,6,8-trifluor-1,4-dihydro-7-(4-methyl-1-piperazinyl)-4-oxo-3-chinolincarbonsäure werden in 15 ml Dimethylsulfoxid mit 1,1 g (10 mmol) 1,4-Diazabicyclo[2.2.2]octan und 660 mg (6 mmol) Thiophenol versetzt und 2 Stunden auf 110°C erhitzt. Die Suspension wird in Wasser eingetragen, der Niederschlag abgesaugt, mit Wasser gewaschen und das Dimethylformamid umkristallisiert.
Ausbeute: 1,1 g (46,7 % der Theorie) 1-Cyclopropyl-6,8-difluor-1,4-dihydro-7-(4-methyl-1-piperazinyl)-4-oxo-5-phenylthio-3-chinolincarbonsäure vom Schmelzpunkt 234-236° (unter Zersetzung).

Beispiel 40

Man arbeitet entsprechend Beispiel 39 mit Mercaptoessigsäuremethylester als Ausgangsverbindung und erhält 1-Cyclopropyl-6,8-difluor-1,4-dihydro-5-(methoxycarbonylmethylthio)-7-(4-methyl-1-piperazinyl)-4-oxo-3-chinolincarbonsäure vom Schmelzpunkt 180-183° (unter Zersetzung).

Beispiel 41

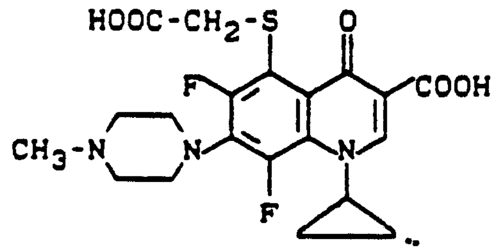

0,4 g (0,86 mmol) 1-Cyclopropyl-6,8-difluor-1,4-dihydro-5-(methoxycarbonylmethylthio)-7-(4-methyl-1-piperazinyl)-4-oxo-3-chinolincarbonsäure werden in 2,7 ml Essigsäure und 1,5 ml Wasser mit 0,3 ml konzentrierter Schwefelsäure versetzt und 2 Stunden auf 150° erhitzt. Die Lösung wird in Eiswasser eingetragen, der ausgefallene Niederschlag abgesaugt, mit Wasser gewaschen und bei 80°/12 mbar getrocknet.
Ausbeute: 390 mg (100 % der Theorie) 5-Carboxymethylthio-1-cyclopropyl-6,8-difluor-1,4-dihydro-7-(4-methyl-1-piperazinyl)-4-oxo-3-chinolincarbonsäure vom Schmelzpunkt ab etwa 265° (unter Zersetzung).

Beispiel 42

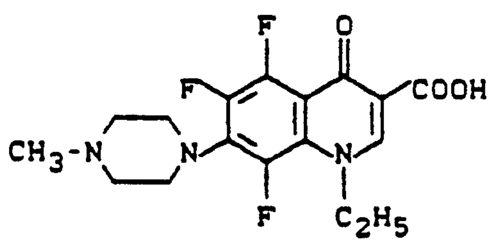

Analog Beispiel 1 setzt man N-Methylpiperazin mit 1-Ethyl-5,6,7,8-tetrafluor-1,4-dihydro-4-oxo-3-chinolincarbonsäure um und erhält 1-Ethyl-5,6,8-trifluor-1,4-dihydro-7-(4-methyl-1-piperazinyl)-4-oxo-3-chinolincarbonsäure vom Schmelzpunkt 214-216° (unter Zersetzung).

Beispiel 43

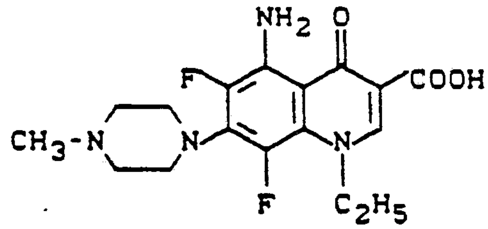

Analog Beispiel 17 wird die Verbindung aus Beispiel 42 mit Ammoniak zu 5-Amino-1-ethyl-6.8-difluor-1,4-dihydro-7-(4-methyl-1-piperazinyl)-4-oxo-3-chinolincarbonsäure vom Schmelzpunkt 246-248° (unter Zersetzung) umgesetzt.

Beispiel 44

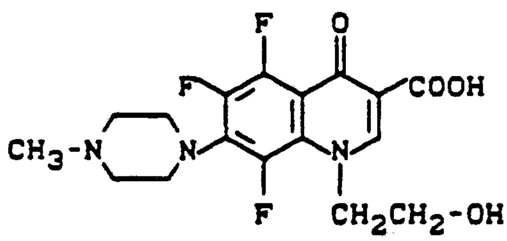

Analog Beispiel 1 setzt man N-Methylpiperazin mit 5,6,7,8-Tetrafluor-1,4-dihydro-1-(2-hydroxyethyl)-4-oxo-3-chinolincarbonsäure um und erhält 5,6,8-Trifluor-1,4-dihydro-1-(2-hydroxyethyl)-7-(4-methyl-1-piperazinyl)-4-oxo-3-chinolincarbonsäure vom Schmelzpunkt 210-211° (unter Zersetzung).

Beispiel 45

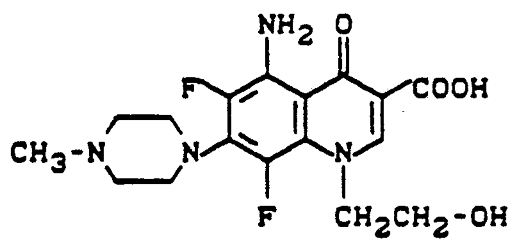

Analog Beispiel 17 wird die Verbindung aus Beispiel 44 mit Ammoniak zu 5-Amino-6,8-difluor-1,4-dihydro-1-(2-hydroxyethyl)-7-(4-methyl-1-piperazinyl)-4-oxo-3-chinolincarbonsäure umgesetzt.

Beispiel 46

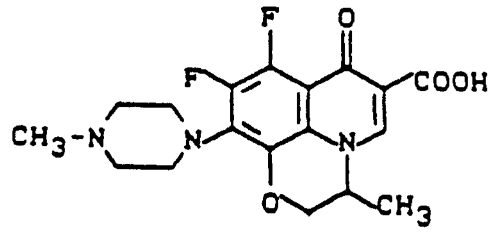

Analog Beispiel 1 setzt man N-Methylpiperazin mit der Verbindung aus Beispiel 4 um und erhält 8,9-Difluor-3-methyl-10-(4-methyl-1-piperazinyl)-7-oxo-7H-pyrido[1,2,3-de][1,4]benzoxazin-6-carbonsäure.

Beispiel 47

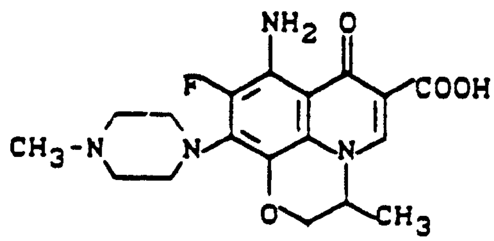

Das Produkt aus Beispiel 46 wird analog Beispiel 17 mit Ammoniak zu 8-Amino-9-fluor-3-methyl-10-(4-methyl-1-piperazinyl)-7-oxo-7H-pyrido[1,2,3-de][1,4]benzoxazon-6-carbonsäure vom Schmelzpunkt 289-290° (unter Zersetzung) umgesetzt.

## Ansprüche

1. 5-Substituierte Chinolon-und Naphthyridoncarbonsäure-Derivate der Formel (I)

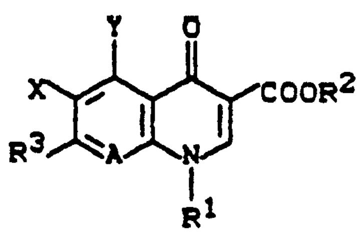

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen je Alkylgruppe, Cycloalkylamino mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,
$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatom oder (5-Methyl-2-oxo-1,3-dioxol-4-yl)-methyl und
$R^3$ für Methyl oder eine cyclische Aminogruppe wie

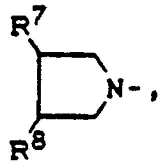, 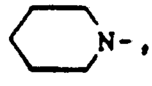, 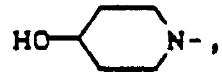, 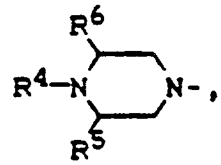,

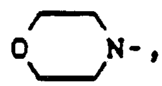, 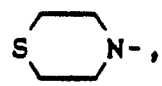, 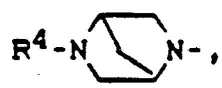, 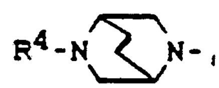,

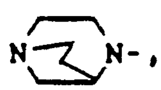, 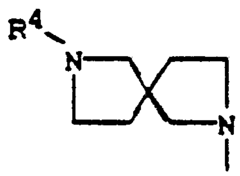, 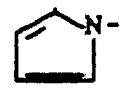, 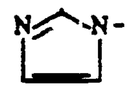,

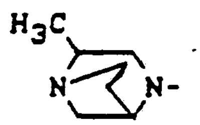

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

-O-$CH_2$-$\underset{|}{C}$H-$CH_3$, -S-$CH_2$-$\underset{|}{C}$H-$CH_3$ oder -$CH_2$-$CH_2$-$\underset{|}{C}$H-$CH_3$

bilden kann,

und deren pharmazeutisch verwendbaren Hydrate und Säureadditionssalze sowie die Alkali-, Erdalkali-, Silber-und Guanidiniumsalze oder zugrundeliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspirol-[4.4]non-2-yl und 7-Methyl (bzw. Ethyl)-2,7-diazaspiro[4.4]non-2-yl bedeutet, und 5-Amino-7-(3-amino-1-pyrrolidinyl)-1-ethyl-6-fluoro-1,4-dihydro-4-oxo-1,8-naphthyridincarbonsäure.

2. 5-Substituierte Chinolon-und Naphthyridoncarbonsäure-Derivate der Formel (I)

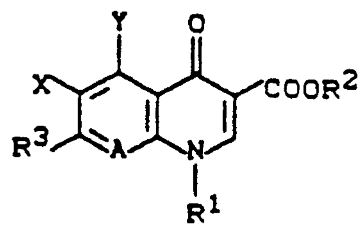

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen je Alkylgruppe, Cycloalkylamino mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,
R' für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl und
$R^3$ für Methyl oder eine cyclische Aminogruppe wie

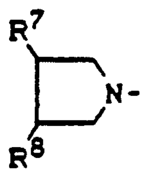, 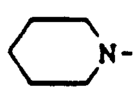, 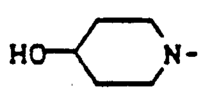, 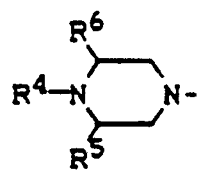,

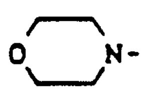, 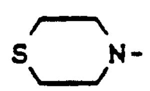, 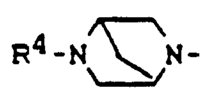, 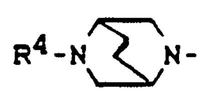,

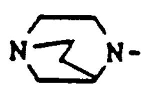, 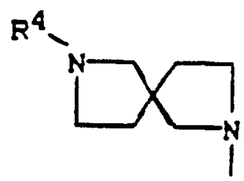, 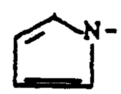, 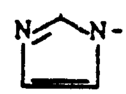,

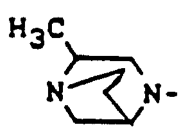

steht, worin
$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,
$R^5$ für Wasserstoff oder Methyl,
$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,
$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,
$R^8$ für Wasserstoff, Methyl, Ethyl, Fluor oder Chlor steht,
A für N oder C-$R^9$ steht, worin
$R^9$ für Wasserstoff, Halogen wie Fluor oder Chlor, Methyl, Cyano oder Nitro steht oder auch gemeinsam mit R' eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze oder zugrundeliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspirol-[4,4]non-2-yl und 7-Methyl (bzw. Ethyl)-2,7-diazaspiro[4,4]non-2-yl bedeutet, und 5-Amino-7-(3-amino-1-pyrrolidinyl)-1-ethyl-6-fluoro-1,4-dihydro-4-oxo-1,8-naphthyridincarbonsäure, sowie ausgenommen Verbindungen, in denen $R^3$ für den Rest

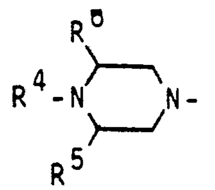

mit $R^4$, $R^5$ und $R^6$ = H oder $C_1$-$C_4$-Alkyl steht, wenn $R^1$ Cyclopropyl, X Fluor, Y Amino, Hydroxy oder $C_1$-$C_3$-Alkoxy und A N bedeutet, $R^9$ für Halogen oder H steht, $R^2$ H bedeutet und deren Säureadditionssalze und Verbindungen, in denen Y für Amino oder Chlor steht, $R^1$ Cyclopropyl und X Fluor bedeutet, A für CF steht sowie $R^3$

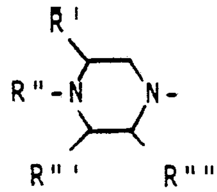

bedeutet,

worin R′ H oder $CH_3$, R″ H, $CH_3$ oder $C_2H_5$, R‴ und R⁗ H oder Methyl bedeuten, und $R^2$ für H, $CH_3$ oder $C_2H_5$ steht.

3. Verbindungen der Formel (I) gemäß Anspruch 1, in der

X für Fluor,

Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen, je Alkylgruppe, Cyclopropylamino, Alkoxyamino mit 1 bis 3 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydroxyamino,

$R^1$ für Ethyl, Isopropyl, Cyclopropyl, Vinyl, 2-Hydroxyethyl, 2-Fluorethyl, Phenyl, 4-Fluorphenyl oder 2,4-Difluorphenyl,

$R^2$ für Wasserstoff, Alkyl mit 1 bis 3 Kohlenstoffatomen, (5-Methyl-2-oxo-1,3-dioxol-4-yl)-methyl,

$R^3$ für eine cyclische Aminogruppe wie

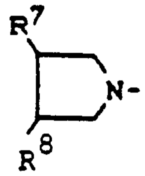, 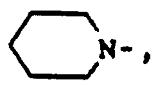, 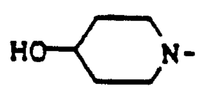, 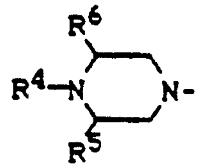,

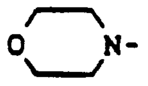, 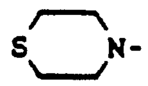, 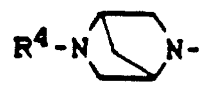, 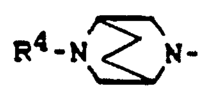,

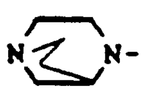, 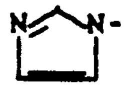, 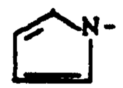, 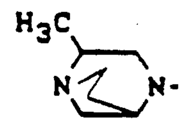

steht, worin

$R^4$ für Wasserstoff, Alkyl mit 1 bis 3 Kohlenstoffatomen, 2-Hydroxyethyl, Allyl, 2-Oxopropyl, 3-Oxobutyl, Phenacyl, Benzyl, 4-Aminobenzyl, (5-Methyl-2-oxo-1,3-dioxol-4-yl)-methyl,

$R^5$ für Wasserstoff oder Methyl,

$R^6$ für Wasserstoff, Alkyl mit 1 bis 2 Kohlenstoffatomen, Phenyl, 4-Fluorphenyl,

$R^7$ für Wasserstoff, Amino, Aminomethyl, Methylaminomethyl, Ethylaminomethyl, Dimethylaminomethyl, Hydroxy oder Hydroxymethyl,

$R^8$ für Wasserstoff oder Methyl steht,

A für N oder C-$R^9$ steht, worin

$R^9$ für Wasserstoff, Halogen wie Fluor oder Chlor steht oder auch gemeinsam mit $R^1$ eine Brücke der Struktur

$-O-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionsalze sowie die Alkali-, Erdalkali-, Silber-und Guanidiniumsalze oder zugrundeliegenden Carbonsäuren, ausgenommen 8-Amino-7-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]-benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl bedeutet, und 5-Amino-7-(3-amino-1-pyrrolidinyl)-1-ethyl-6-fluor-1,4-dihydro-4-oxo-1,8-naphthyridin-3-carbonsäure.

4. Verbindungen der Formel (I) gemäß Anspruch 1, in der

X für Fluor,

Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen je Alkylgruppe, Cyclopropylamino, Methoxyamino, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio,

$R^1$ für Ethyl, Cyclopropyl, 2-Hydroxyethyl, 4-Fluorphenyl oder 2,4-Difluorphenyl,

$R^2$ für Wasserstoff, Alkyl mit 1 bis 2 Kohlenstoffatomen

$R^3$ für eine cyclische Aminogruppe wie

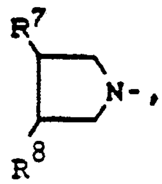, 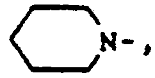, 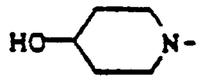, 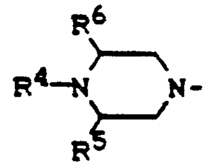,

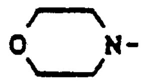, 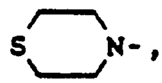, 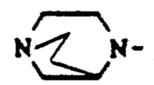, 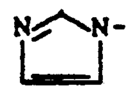, 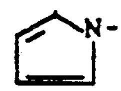,

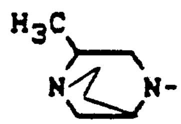

steht, worin

$R^4$ für Wasserstoff, Alkyl mit 1 bis 3 Kohlenstoffatomen, 2-Hydroxyethyl, 2-Oxopropyl, 3-Oxobutyl, Phenacyl, 4-Aminobenzyl

$R^5$ für Wasserstoff oder Methyl,

$R^6$ für Wasserstoff, Methyl Phenyl, 4-Fluorphenyl,

$R^7$ für Wasserstoff, Amino, Aminomethyl, Methylaminomethyl, Ethylaminomethyl, Hydroxy oder Hydroxymethyl,

$R^8$ für Wasserstoff steht,

A für N oder C-$R^9$ steht, worin

$R^9$ für Wasserstoff, Halogen wie Fluor oder Chlor steht oder auch gemeinsam mit R' eine Brücke der Struktur

$-O-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze der zugrundeliegenden Carbonsäuren ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl oder 3-(Ethylaminoethyl)-1-pyrrolidinyl bedeutet, und 5-Amino-7-(3-amino-1-pyrrolidinyl)-1-ethyl-6-fluor-1,4-dihydro-4-oxo-1,8-naphthyridin-3-carbonsäure.

5. Verbindungen aus der Gruppe bestehend aus 1-Cyclopropyl-5,6-difluor-1,4-dihydro-7-(3-hydroxy-1-pyrrolidinyl)-4-oxo-3-chinolincarbonsäure, 1-Cyclopropyl-5,6,8-trifluor-1,4-dihydro-7-(3-hydroxy-1-pyrrolidinyl)-4-oxo-3-chinolincarbonsäure,

1-Cyclopropyl-5,6-difluor-1,4-dihydro-7-(3-hydroxy-1-pyrrolidinyl)-4-oxo-3-chinolincarbonsäure, 1-Cyclopropyl-7-(1,4-diazabicyclo[3,2,1]oct-4-yl)-5,6,8-trifluor-1,4-dihydro-4-oxo-3-chinolincarbonsäure,

1-Cyclopropyl-7-(1,4-diazabicyclo[3,2,1]oct-4-yl)-5,6-difluor-1,4-dihydro-4-oxo-1,8-naphthyridin-3-carbonsäure,

1-Cyclopropyl-5,6,8-trifluor-1,4-dihydro-7-(2-methyl-1,4-diazabicyclo[3,2,1]oct-4-yl)-4-oxo-3-chinolincarbonsäure,

1-Cyclopropyl-5,6,8-trifluor-1,4-dihydro-7-(8-methyl-3,8-diazabicyclo[3,2,1]oct-3-yl)-4-oxo-3-chinolincarbonsäure,

1-Cyclopropyl-5,6,8-trifluor-1,4-dihydro-4-oxo-7-(1-piperazinyl)-3-chinolincarbonsäure,

1-Cyclopropyl-5,6,8-trifluor-1,4-dihydro-4-oxo-7-(4-methyl-1-piperazinyl)-3-chinolincarbonsäure,

1-Cyclopropyl-5,6,8-trifluor-1,4-dihydro-4-oxo-7-(4-ethyl-1-piperazinyl)-3-chinolincarbonsäure,

1-Cyclopropyl-5,6,8-trifluor-1,4-dihydro-7-[4-(2-hydroxyethyl)-1-piperazinyl]-4-oxo-3-chinolincarbonsäure,

1-Cyclopropyl-5,6,8-trifluor-1,4-dihydro-4-oxo-7-(3-methyl-1-piperazinyl)-3-chinolincarbonsäure,

1-Cyclopropyl-5,6,8-trifluor-1,4-dihydro-4-oxo-7-(3,5-dimethyl-1-piperazinyl)-3-chinolincarbonsäure,

1-Cyclopropyl-5,6,8-trifluor-1,4-dihydro-4-oxo-7-morpholino-3-chinolincarbonsäure,
1-Cyclopropyl-5,6,8-trifluor-1,4-dihydro-4-oxo-7-thiomorpholino-3-chinolincarbonsäure,
1-Cyclopropyl-5,6,8-trifluor-1,4-dihydro-4-oxo-7-(4-isopropyl-1-piperazinyl)-3-chinolincarbonsäure,
7-(4-Allyl-1-piperazinyl)-1-cyclopropyl-5,6,8-trifluor-1,4-dihydro-4-oxo-3-chinolincarbonsäure,
1-Cyclopropyl-5,6,8-trifluor-1,4-dihydro-4-oxo-7-[4-(2-oxopropyl)-1-piperazinyl]-3-chinolincarbonsäure,
1-Cyclopropyl-5,6,8-trifluor-1,4-dihydro-4-oxo-7-[4-(3-oxobutyl)-1-piperazinyl]-3-chinolincarbonsäure,
1-Cyclopropyl-5,6,8-trifluor-1,4-dihydro-4-oxo-7-(3-phenyl-1-piperazinyl)-3-chinolincarbonsäure,
1-Cyclopropyl-5,6,8-trifluor-1,4-dihydro-7-(1-imidazolyl)-4-oxo-3-chinolincarbonsäure,
1-Cyclopropyl-5,6,8-trifluor-1,4-dihydro-4-oxo-7-(1-pyrrolyl)-3-chinolincarbonsäure.

6. Verbindungen aus der Gruppe bestehend aus
5-Amino-7-[3-(aminomethyl)-1-pyrrolidinyl]-1-cyclopropyl-6,8-difluor-1,4-dihydro-4-oxo-3-chinolincarbonsäure,
5-Amino-1-cyclopropyl-7-[3-[(methylamino)methyl]-1-pyrrolidinyl]-6,8-difluor-1,4-dihydro-4-oxo-3-chinolincarbonsäure,
5-Amino-1-cyclopropyl-7-[3-[(ethylamino)methyl]-1-pyrrolidinyl]-6,8-difluor-1,4-dihydro-4-oxo-3-chinolincarbonsäure,
5-Amino-7-[3-amino-1-pyrrolidinyl]-1-cyclopropyl-6,8-difluor-1,4-dihydro-4-oxo-3-chinolincarbonsäure,
5-Amino-1-cyclopropyl-7-[3-(ethylamino)-1-pyrrolidinyl]-6,8-difluor-1,4-dihydro-4-oxo-3-chinolincarbonsäure,
5-Amino-1-cyclopropyl-6,8-difluor-1,4-dihydro-7-[3-[[(1-methylethyl)amino]methyl]-1-pyrrolidinyl]-4-oxo-3-chinolincarbonsäure,
5-Amino-7-[3-(aminomethyl)-1-pyrrolidinyl]-1-cyclopropyl-1,4-dihydro-6-fluor-4-oxo-1,8-naphthyridin-3-carbonsäure,
5-Amino-1-cyclopropyl-7-[3-[(ethylamino)methyl]-1-pyrrolidinyl]-1,4-dihydro-6-fluor-4-oxo-1,8-naphthyridin-3-carbonsäure,
5-Amino-7-[3-amino-1-pyrrolidinyl]-1-cyclopropyl-1,4-dihydro-6-fluor-4-oxo-1,8-naphthyridin-3-carbonsäure,
5-Amino-7-[3-(aminomethyl)-1-pyrrolidinyl]-1-ethyl-6-fluor-1,4-dihydro-4-oxo-1,8-naphthyridin-3-carbonsäure,
5-Amino-7-[3-(aminomethyl)-1-pyrrolidinyl]-1-ethyl-6,8-difluor-1,4-dihydro-4-oxo-3-chinolincarbonsäure,
5-Amino-7-[3-(aminomethyl)-1-pyrrolidinyl]-6,8-difluor-1-(2-fluoroethyl)-1,4-dihydro-4-oxo-3-chinolincarbonsäure,
5-Amino-7-[3-(aminomethyl)1-pyrrolidinyl]-6,8-difluor-1-ethenyl-1,4-dihydro-4-oxo-3-chinolincarbonsäure,
5-Amino-1-ethyl-7-[3-[(ethylamino)methyl]-1-pyrrolidinyl]-6-fluor-1,4-dihydro-4-oxo-1,8-naphthyridin-3-carbonsäure,
5-Amino-1-ethyl-7-[3-[(ethylamino)methyl]-1-pyrrolidinyl]-6,8-difluor-1,4-dihydro-4-oxo-3-chinolincarbonsäure,
5-Amino-7-[3-[(ethylamino)methyl]-1-pyrrolidinyl]-6,8-difluor-1-(2-fluorethyl)-1,4-dihydro-4-oxo-3-chinolincarbonsäure,
5-Amino-1-ethenyl-7-[3-[(ethylamino)methyl-1-pyrrolidinyl]-6,8-difluor-1,4-dihydro-4-oxo-3-chinolincarbonsäure,
5-Amino-1-ethyl-6-fluor-1,4-dihydro-7-[3-[[(1-methylethyl)-amino]methyl]-1-pyrrolidinyl]-4-oxo-1,8-naphthyridin-3-carbonsäure,
5-Amino-1-ethyl-7-[3-[(1-methylethyl)aminomethyl]-1-pyrrolidinyl]-6,8-difluor-1,4-dihydro-4-oxo-3-chinolincarbonsäure,
5-Amino-1-ethyl-6-fluor-1,4-dihydro-7-(7-methyl-2,7-diazaspiro[4.4]non-2-yl)-4-oxo-1,8-naphthyridin-3-carbonsäure,
5-Amino-1-ethyl-6,8-difluor-1,4-dihydro-7-(7-ethyl-2,7-diazaspiro[4.4]non-2-yl)-4-oxo-3-chinolincarbonsäure,
5-Amino-1-cyclopropyl-6,8-difluor-1,4-dihydro-7-(7-methyl-2,7-diazaspiro[4.4]non-2-yl]-4-oxo-3-chinolincarbonsäure,
5-Amino-7-(3-amino-1-pyrrolidinyl)-1-ethyl-6,8-difluor-1,4-dihydro-4-oxo-3-chinolincarbonsäure, 5-Amino-1-cyclopropyl-6-fluor-1,4-dihydro-7-(3-hydroxy-1-pyrrolidinyl)-4-oxo-3-chinolincarbonsäure,
5-Amino-1-cyclopropyl-6-fluor-1,4-dihydro-7-(3-hydroxy-1-pyrrolidinyl)-4-oxo-3-chinolincarbonsäure,
5-Amino-1-cyclopropyl-7-(1,4-diazabicyclo[3.2.1]oct-4-yl)-6-fluor-1,4-dihydro-4-oxo-1,8-naphthyridin-3-carbonsäure,
5-Amino-1-cyclopropyl-6,8-difluor-1,4-dihydro-7-(2-methyl-1,4-diazabicyclo[3.2.1]oct-4-yl)-4-oxo-3-chinolincarbonsäure,
5-Amino-1-cyclopropyl-6,8-difluor-1,4-dihydro-7-(8-methyl-3,8-diazabicyclo[3.2.1]oct-3-yl)-4-oxo-3-chinolincarbonsäure,
5-Amino-1-cyclopropyl-6,8-difluor-1,4-dihydro-4-oxo-7-(1-piperazinyl)-3-chinolincarbonsäure,
5-Amino-1-cyclopropyl-6,8-difluor-1,4-dihydro-4-oxo-7-thiomorpholino-3-chinolincarbonsäure,
5-Amino-1-cyclopropyl-6,8-difluor-1,4-dihydro-4-oxo-7-(4-isopropyl-1-piperazinyl)-3-chinolincarbonsäure,

5-Amino-7-(4-allyl-1-piperazinyl)-1-cyclopropyl-6,8-difluor-1,4-dihydro-4-oxo-3-chinolincarbonsäure,
5-Amino-1-cyclopropyl-6,8-difluor-1,4-dihydro-4-oxo-7-[4-(2-oxopropyl)-1-piperazinyl]-3-chinolincarbonsäure,
5-Amino-1-cyclopropyl-6,8-fluor-1,4-dihydro-4-oxo-7-[4-(3-oxobutyl)-1-piperazinyl]-3-chinolincarbonsäure,
5-Amino-1-cyclopropyl-6,8-fluor-1,4-dihydro-4-oxo-7-(1-pyrrolyl)-3-chinolincarbonsäure,
5-Amino-7-[4-(4-aminobenzyl)-1-piperazinyl]-1-cyclopropyl-6,8-difluor-1,4-dihydro-4-oxo-3-chinolincarbonsäure,
5-Amino-1-cyclopropyl-6,8-difluor-1,4-dihydro-4-oxo-7-(4-phenacyl-1-piperazinyl)-3-chinolincarbonsäure,
S-8-Amino-9-fluor-3-methyl-10-(4-methyl-1-piperazinyl)-7-oxo-7H-pyrido[1,2,3-de][1,4]benzoxazin-6-carbonsäure.

7. 5-Substituierte Chinolon-und Naphthyridoncarbonsäure-Derivate der Formel (I)

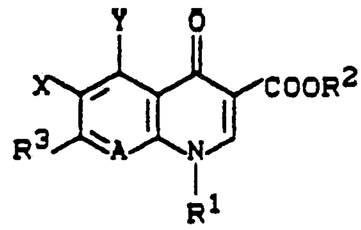

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen, je Alkylgruppe, Cycloalkylamino, mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,
$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl und
$R^3$ für Methyl oder eine cyclische Aminogruppe wie

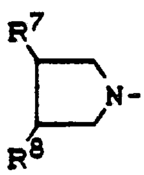 , 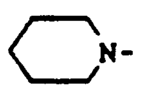 , 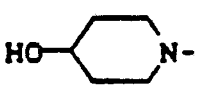 , 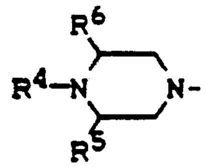 ,

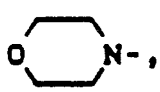 , 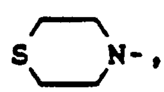 , 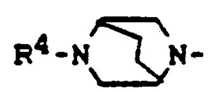 , 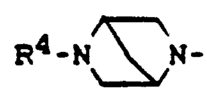 ,

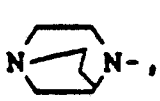 , 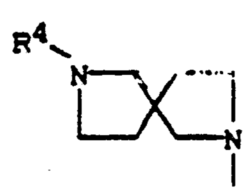 , 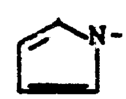 , 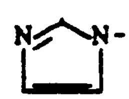 ,

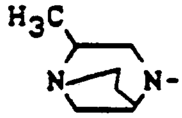

steht, worin
$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze der zugrundliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrid[1,2,3-de][1,4]benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspiro[4.4]non-2-yl und 7-Methyl(bzw. Ethyl)-2,7-diazaspiro[4.4]non-2-yl bedeutet, und 5-Amino-7-(3-amino-1-pyrrolidinyl)-1-ethyl-6-fluoro1,4-dihydro-4-oxo-1,8-naphthyridincarbonsäure zur Anwendung in einem Verfahren zur therapeutischen Behandlung des menschlichen oder tierischen Körpers.

8. Verfahren zur Herstellung von 5-substituierten Chinolon-und Naphthyridoncarbonsäure-Derivaten der Formel (I)

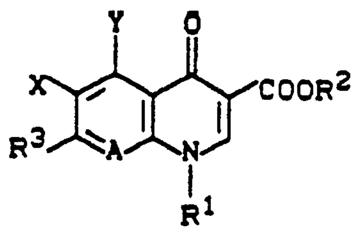

(I),

in welcher

X für Fluor oder Chlor,

Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen, je Alkylgruppe, Cycloalkylamino, mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,

$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,

$R^2$ für Wasserstoff, Alkyl mit 1 bis 4 Kohlenstoffatomeno der (5-Methyl-2-oxo-1,3-dioxol-4-yl)-methyl und

$R^3$ für Methyl oder eine cyclische Aminogruppe wie

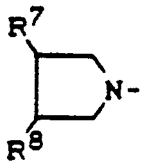, 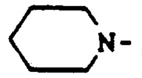, 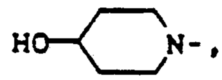, 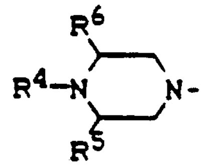,

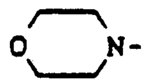, 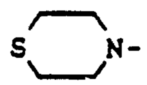, 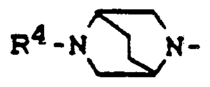, 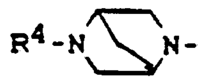,

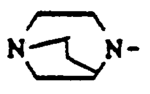, 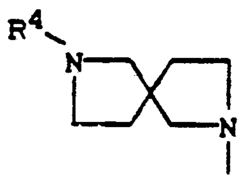, 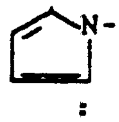, 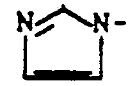,

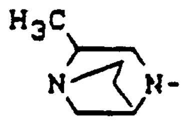

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und deren pharmazeutisch verwendbaren Hydrate und Säureadditionssalze sowie die Alkali-, Erdalkali-, Silber-und Guanidiniumsalze der zugrundliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrid[1,2,3-de][1,4]benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspiro[4.4]non-2-yl und 7-Methyl(bzw. Ethyl)-2,7-diazaspiro[4.4]non-2-yl bedeutet, und 5-Amino-7-(3-amino-1-pyrrolidinyl)-1-ethyl-6-fluoro-1,4-dihydro-4-oxo-1,8-naphthyridincarbonsäure, dadurch gekennzeichnet, daß man Verbindungen der Formel (II)

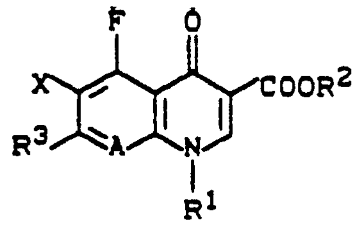

(II),

in welcher
X, R', $R^2$, $R^3$ und A die oben angegebene Bedeutung haben,
mit Verbindungen der Formel (III)

Y-H (III),

in welcher
Y die oben angegebene Bedeutung hat,
gegebenenfalls in Gegenwart von Säurefängern umsetzt.

9. Arzneimittel, enthaltend 5-substituierte Chinolon-und Naphthyridoncarbonsäure-Derivate der Formel (I)

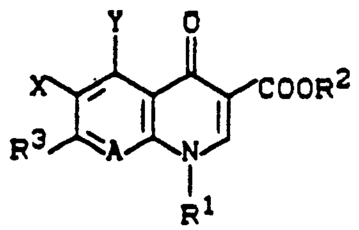

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen, je Alkylgruppe, Cycloalkylamino, mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,
R' für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,
$R^2$ für Wasserstoff, Alkyl mit 1 bis 4 Kohlenstoffatomen oder (5-Methyl-2-oxo-1,3-dioxol-4-yl)-methyl und
$R^3$ für Methyl oder eine cyclische Aminogruppe wie

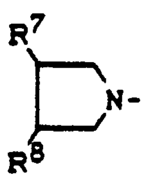 , 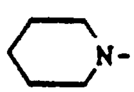 , 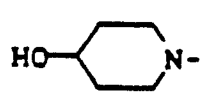 , 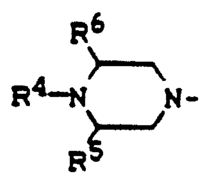 ,

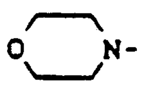, 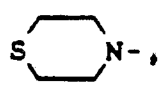, 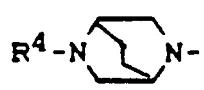, 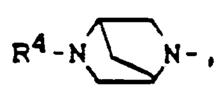,

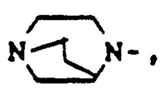, 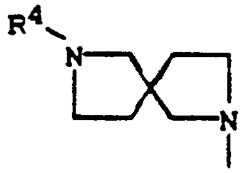, 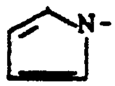, 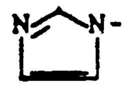,

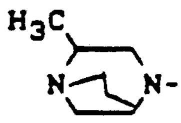

steht, worin
$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,
$R^5$ für Wasserstoff oder Methyl,
$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,
$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,
$R^8$ für Wasserstoff, Methyl, Ethyl, Fluor oder Chlor steht,
A für N oder C-$R^9$ steht, worin

$R^9$ für Wasserstoff, Halogen wie Fluor oder Chlor, Methyl, Cyano oder Nitro steht oder auch gemeinsam mit R' eine Brücke der Struktur
$-O-CH_2-\underset{|}{C}H-CH_3$, $-S-CH_2-\underset{|}{C}H-CH_3$ oder $-CH_2-CH_2-\underset{|}{C}H-CH_3$

bilden kann,
und deren pharmazeutisch verwendbaren Hydrate und Säureadditionssalze sowie die Alkali-, Erdalkali-, Silber-und Guanidiniumsalze der zugrundliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3-de][1,4]benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspiro-[4.4]non-2-yl und 7-Methyl(bzw. Ethyl)-2,7-diazaspiro[4.4]non-2-yl bedeutet, und 5-Amino-7-(3-amino-1-pyrrolidinyl)-1-ethyl-6-fluoro-1,4-dihydro-4-oxo-1,8-naphthyridincarbonsäure.

10. Verwendung von 5-substituierten Chinolon-und Naphthyridoncarbonsäure-Derivaten der Formel (I)

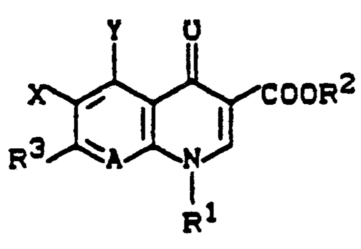

(I),

in welcher
X für Fluor oder Chlor,
Y für Amino, gegebenenfalls durch Amino, Hydroxy oder Methoxy substituiertes Alkylamino mit 1 bis 4 Kohlenstoffatomen, Dialkylamino mit 1 bis 4 Kohlenstoffatomen, je Alkylgruppe, Cycloalkylamino, mit 3 bis 6 Kohlenstoffatomen, Alkoxyamino mit 1 bis 4 Kohlenstoffatomen, Hydroxy, Alkoxy mit 1 bis 4 Kohlenstoffatomen, Mercapto, gegebenenfalls durch Ethoxycarbonyl, Carboxy oder Hydroxy substituiertes Alkylthio mit 1 bis 4 Kohlenstoffatomen, Phenylthio, Hydrazino, Hydroxyamino, Methoxyamino, Chlor,

$R^1$ für Methyl, Ethyl, Propyl, Isopropyl, Cyclopropyl, Vinyl, 2-Hydroxyethyl, 2-Fluorethyl, Methoxy, Amino, Dimethylamino, Ethylamino, Phenyl, 4-Fluorphenyl oder 2,4-Difluorphenyl,

$R^2$ für Wasserstoff, Alkyl mit 1 bis 4 Kohlenstoffatomeno der (5-Methyl-2-oxo-1,3-dioxol-4-yl)-methyl und

$R^3$ für Methyl oder eine cyclische Aminogruppe wie

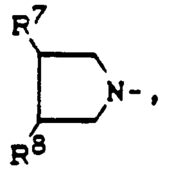 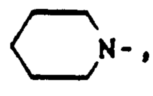 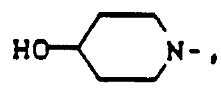 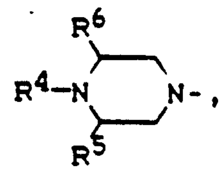

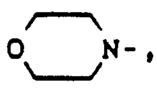 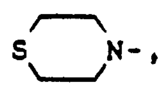 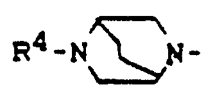 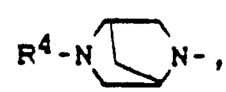

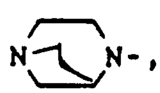 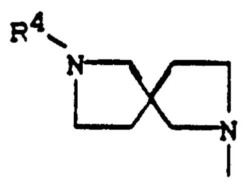 , 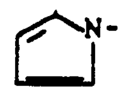 , 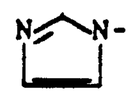 ,

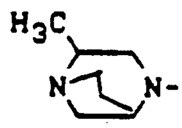

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, Benzyl, 4-Aminobenzyl oder (5-Methyl-2-oxo-1,3-dioxol-4-yl)-methyl,

$R^5$ für Wasserstoff oder Methyl,

$R^6$ für Wasserstoff, Alkyl mit 1 bis 4 Kohlenstoffatomen, gegebenenfalls substituiertes, insbesondere durch Fluor substituiertes Phenyl oder Benzyloxymethyl,

$R^7$ für Wasserstoff, Amino, Methylamino, Ethylamino, Aminomethyl, Methylaminomethyl, Ethylaminomethyl, Dimethylaminomethyl, Isopropylaminomethyl, Hydroxy oder Hydroxymethyl,

$R^8$ für Wasserstoff, Methyl, Ethyl, Fluor oder Chlor steht,

A für N oder C-$R^9$ steht, worin

$R^9$ für Wasserstoff, Halogen wie Fluor oder Chlor, Methyl, Cyano oder Nitro steht oder auch gemeinsam mit $R^1$ eine Brücke der Struktur

$-O-CH_2-\underset{|}{C}H-CH_3$, $-S-CH_2-\underset{|}{C}H-CH_3$ oder $-CH_2-CH_2-\underset{|}{C}H-CH_3$

bilden kann,

und von deren pharmazeutisch verwendbaren Hydraten und Säureadditionssalzen sowie den Alkali-, Erdalkali-, Silber-und Guanidiniumsalzen der zugrundeliegenden Carbonsäuren, ausgenommen 8-Amino-9-fluor-3-methyl-10-(4-methyl-1-piperazinyl)-7-oxo-2,3-dihydro-7H-pyrido[1,2,3-de][1,4]benzoxazin-6-carbonsäure, 1-Ethyl-5-amino-6,8-difluor-7-(1-piperazinyl)-4-oxo-1,4-dihydro-3-chinolincarbonsäure, 1-Ethyl-5-amino-6,8-difluor-7-(4-methyl-1-piperazinyl)-4-oxo-1,4-dihydro-3-chinolincarbonsäure sowie ausgenommen 8-Amino-9-fluor-3-methyl-10-(heterocyclyl)-7-oxo-2,3-dihydro-7H-pyrido[1,2,3=de][1,4]benzoxazin-6-carbonsäuren und 1-Ethyl-5-amino-6,8-difluor-7-(heterocyclyl)-4-oxo-1,4-dihydro-3-chinolincarbonsäuren, in denen Heterocyclyl 3-Amino-1-pyrrolidinyl, 3-(Aminomethyl)-1-pyrrolidinyl, 3-(Ethylaminomethyl)-1-pyrrolidinyl, 2,7-Diazaspiro[4.4]non-2-yl und 7-Methyl(bzw. Ethyl)-2,7-diazaspiro[4.4]non-2-yl bedeutet, und 5-Amino-7-(3-amino-1-pyrrolidinyl)-1-ethyl-6-fluoro-1,4-dihydro-4-oxo-1,8-naphthyridincarbonsäure zur Herstellung von Arzneimitteln.

11. Verwendung der Verbindungen nach Anspruch 1 zur Herstellung von veterinärmedizinischen Arzneimitteln und Tierfuttermitteln.

Europäisches Patentamt

# EUROPÄISCHER RECHERCHENBERICHT

Nummer der Anmeldung

EP 88 10 4452

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,P | EP-A-0 221 463 (DAINIPPON PHARMACEUTICAL CO., LTD.)<br>* Beispiele 1-10,13-15 * | 1-10 | C 07 D 215/56<br>C 07 D 471/04<br>C 07 D 498/06<br>A 61 K 31/47<br>A 61 K 31/44<br>A 61 K 31/535//<br>(C 07 D 471/04<br>C 07 D 221:00<br>C 07 D 221:00 )<br>(C 07 D 498/06<br>C 07 D 265:00<br>C 07 D 221:00 ) |
| X,P | EP-A-0 242 789 (DAINIPPON PHARMACEUTICAL CO., LTD.)<br>* Ansprüche 1,4,12,16; Beispiele 10-23 * | 1-10 | |
| X | EP-A-0 115 334 (OTSUKA PHARMACEUTICAL CO., LTD.)<br>* Beispiele 3-9; Anspruch 6 * | 1-4,7,9,10 | |
| X | PATENT ABSTRACTS OF JAPAN, Band 9, Nr. 331 (C-321)[2054], 25. Dezember 1985; & JP - A - 60 161 987 (OTSUKA SEIYAKU K.K.) 23.08.1985 | 1-4,7,9,10 | |
| X,P | EP-A-0 226 961 (WARNER-LAMBERT CO.)<br>* Ansprüche 1-4; Beispiele 1-4 * | 1-4,7,9,10 | **RECHERCHIERTE SACHGEBIETE (Int. Cl.4)** |
| X,P | CHEMICAL ABSTRACTS, Band 108, Nr. 21, 23. Mai 1988, Seite 691, Zusammenfassung Nr. 186591v, Columbus, Ohio, US; & JP - A - 62 226 962 (DAINIPPON PHARMACEUTICAL CO., LTD.) | 1-4,7,9,10 | C 07 D 215/00<br>C 07 D 471/00<br>C 07 D 498/00 |
| X,P | CHEMICAL ABSTRACTS, Band 107, Nr. 1, 6. Juli 1987, Seite 668, Zusammenfassung Nr. 7219q; & JP - A - 62 33188 (OTSUKA PHARMACEUTICAL CO., LTD.) (Kat. X) | 1-4,7,9,10 | |
| Y,D | EP-A-0 172 651 (WARNER-LAMBERT CO.)<br>* Ansprüche 1-9,11 *<br>-/- | 1-4,7,9,10 | |

Der vorliegende Recherchenbericht wurde für alle Patentansprüche erstellt

| Recherchenort | Abschlußdatum der Recherche | Prüfer |
|---|---|---|
| BERLIN | 07-07-1988 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 88 10 4452

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 98, Nr. 9, 28. Februar 1983, Seite 635, Zusammenfassung Nr. 72117q, Columbus, Ohio, US; & JP - A - 57 149 286 (DAIICHI SEIYAKU CO. LTD.) (Kat. Y,D)<br>--- | 1-4,7,9,10 | |
| Y | CHEMICAL ABSTRACTS, Band 100, Nr. 7, 13. Februar 1984, Seite 570, Zusammenfassung 51467y, Columbus, Ohio, US; & JP - A - 58 174 367 (TANABE SEIYAKU CO., LTD.) (Kat. Y,D)<br>--- | 1-4,7,9,10 | |
| Y | CHEMICAL ABSTRACTS, Band 106, Nr. 1, 5. Januar 1987, Seite 459; Zusammenfassung Nr. 4879y, Columbus, Ohio, US; & ZA - 85 02369 (ABBOTT LABORARTORIES)<br>--- | 1-4,7,9,10 | |
| A,D | EP-A-0 202 763 (WARNER-LAMBERT CO.)<br>----- | 1 | |
| | | | RECHERCHIERTE SACHGEBIETE (Int. Cl.4) |

Der vorliegende Recherchenbericht wurde für alle Patentansprüche erstellt

| Recherchenort | Abschlußdatum der Recherche | Prüfer |
|---|---|---|
| BERLIN | 07-07-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX

DATE: 09 SEP 88 AT 11:05:58

DEPARTMENT: ELECTRONIC:PRINTING*

JOB ID: 253 REPORT NO. 5

FILE ID:

INPUT PROCESSING TIME: 00:00:16

OUTPUT PROCESSING TIME: 00:00:53

REPORT COMPLETION CODE: 0

| | | | |
|---|---|---|---|
| PAGES TO BIN: | 45 | | |
| PAGES TO TRAY: | 0 | | |
| PAPER PATH HOLES: | 0 | | |
| LINES PRINTED: | 2966 | GRAPHIC PAGES PRINTED: | 1 |
| TAPE MOUNTS: | 1 | GRAPHIC EXCEPTION CODE: | 0 |
| BLOCKS READ: | 38 | GRAPHIC IMAGES READ: | 0 |
| BLOCKS SKIPPED: | 0 | GRAPHIC IMAGES MOVED: | 0 |
| RECORDS READ: | 2937 | | |
| DJDE RECORDS READ: | 5 | | |
| MAXIMUM COPY COUNT: | 1 | | |
| OVERPRINTS: | 2888 | | |
| COLLATE: | YES | | |
| SF/MF: | MULTI | | |
| SIMPLEX/DUPLEX: | BOTH | | |

JDE,JDL USED: EP,XICS

ACCTINFO:

INITIAL FONT LIST: Z0408P

INITIAL FORM LIST: -NONE

INITIAL CME LIST: -NONE

XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX
XEROX EPS XEROX EPS XEROX EPS XEROX EPS XEROX EPS XEROX EPS XEROX